# EUROPÄISCHE PATENTSCHRIFT

(11) **EP 2 271 584 B1**
(45) Veröffentlichungstag und Bekanntmachung des Hinweises auf die Patenterteilung: **15.05.2013**
(21) Anmeldenummer: 09727777.6
(22) Anmeldetag: 13.03.2009
(51) Int. Cl.: C01B 21/22, B01D 53/14

(54) **VERFAHREN ZUR REINIGUNG VON N2O**
PROCESS FOR PURIFYING N2O
PROCÉDÉ D ÉPURATION DU N2O

(30) Priorität: 02.04.2008 EP 08153952
(43) Veröffentlichungstag der Anmeldung: 12.01.2011
(73) Patentinhaber: BASF SE, 67056 Ludwigshafen (DE)
(72) Erfinder: RÖSSLER-FEIGEL, Beatrice, 67256 Weisenheim am Sand (DE); TELES, Joaquim Henrique, 67166 Otterstadt (DE); BAUMANN, Dieter, 67346 Speyer (DE)
(74) Vertreter: Lutz, Andreas
(86) Internationale Anmeldenummer: PCT/EP2009/052991
(87) Internationale Veröffentlichungsnummer: WO 2009/121706

(56) Entgegenhaltungen:
- WO-A-2006/032502
- DE-A1-102005 055 588
- MIKODA JIRI: "Separation of dinitrogen monoxide [from gas mixtures]" CAPLUS,, 12. Mai 1984 (1984-05-12), XP002481315

## Beschreibung

Die vorliegende Erfindung betrifft ein Verfahren zur Reinigung eines Gasgemischs enthaltend Distickstoffmonoxid mindestens umfassend das Behandeln eines Gasgemischs G-0 enthaltend Distickstoffmonoxid unter Erhalt eines Gasgemischs G-A mindestens umfassend die Absorption des Gasgemischs G-0 in einem Lösungsmittelgemisch LM-I unter Erhalt eines Abgasstroms und einer Zusammensetzung Z-A und die Desorption eines Gasgemischs G-1 aus der Zusammensetzung Z-A unter Erhalt eines Lösungsmittelgemischs LM-I', anschließende Kondensation des Gasgemischs G-A unter Erhalt einer flüssigen Zusammensetzung Z-1 enthaltend Distickstoffmonoxid und einem gasförmigen Gemisch G-K, wobei das gasförmige Gemisch G-K in das Verfahren zurückgeführt wird.

Im Rahmen der vorliegenden Erfindung ist es auch möglich, das die Zusammensetzung des Gasgemischs G-A der des Gasgemischs G-1 entspricht.

Aus dem Stand der Technik sind verschiedene Herstellungsverfahren und Reinigungsverfahren für Distickstoffmonoxid bekannt. Es ist ebenfalls bekannt, dass Distickstoffmonoxid beispielsweise als Oxidationsmittel für Olefine eingesetzt werden kann.

So offenbart die WO 98/25698 ein Verfahren zur Herstellung von Distickstoffmonoxid durch katalytische Partialoxidation von NH₃ mit Sauerstoff. Dabei wird gemäß der WO 98/25698 ein Katalysator aus Manganoxid, Bismutoxid und Aluminiumoxid eingesetzt, der mit hoher Selektivität zu Distickstoffmonoxid führt. Ein ähnliches Katalysatorsystem wird auch in einer wissenschaftlichen Arbeit näher beschrieben (Noskov et al., Chem. Eng. J. 91 (2003) 235-242). Gemäß der US 5,849,257 wird ebenfalls ein Verfahren zur Herstellung von Distickstoffmonoxid durch Oxidation von Ammoniak offenbart. Dabei findet die Oxidation in Gegenwart eines Kupfer-Manganoxid Katalysators statt.

Gemäß dem in der WO 00/01654 offenbarten Verfahren wird Distickstoffmonoxid hergestellt, indem ein Gasstrom enthaltend NOₓ und Ammoniak reduziert wird.

Die Oxidation einer olefinischen Verbindung zu einem Aldehyd oder einem Keton mittels Distickstoffmonoxid ist beispielsweise beschrieben in der GB 649,680 oder der dazu äquivalenten US 2,636,898. In beiden Schriften wird ganz allgemein offenbart, dass die Oxidation prinzipiell in Anwesenheit eines geeigneten Oxidationskatalysators erfolgen kann.

In den neueren wissenschaftlichen Artikeln von G. I. Panov et al., "Non-Catalytic Liquid Phase Oxidation of Alkenes with Nitrous Oxide. 1. Oxidation of Cyclohexene to Cyclohexanone", React. Kinet. Catal. Lett. Vol. 76, No. 2 (2002) S. 401-405, und K. A. Dubkov et al., "Non-Catalytic Liquid Phase Oxidation of Alkenes with Nitrous Oxide. 2. Oxidation of Cyclopentene to Cyclopentanone", React. Kinet. Catal. Lett. Vol. 77, No. 1 (2002) S. 197-205 werden ebenfalls Oxidationen von olefinischen Verbindungen mit Distickstoffmonoxid beschrieben. Auch ein wissenschaftlicher Artikel "Liquid Phase Oxidation of Alkenes with Nitrous Oxide to Carbonyl Compounds" von E. V. Starokon et al. in Adv. Synth. Catal. 2004, 346, 268 - 274 beinhaltet eine mechanistische Studie der Oxidation von Alkenen mit Distickstoffmonoxid in flüssiger Phase.

Die Synthese von Carbonylverbindungen aus Alkenen mit Distickstoffmonoxid wird auch in verschiedenen internationalen Patentanmeldungen beschrieben. So offenbart die WO 03/078370 ein Verfahren zur Herstellung von Carbonylverbindungen aus aliphatischen Alkenen mit Distickstoffmonoxid. Die Umsetzung wird bei Temperaturen im Bereich von 20 bis 350°C und Drücken von 0,01 bis 100 atm durchgeführt. Die WO 03/078374 offenbart ein entsprechendes Verfahren zur Herstellung von Cyclohexanon. Gemäß der WO 03/078372 werden cyclische Ketone mit 4 bis 5 C-Atomen hergestellt. Gemäß der WO 03/078375 werden unter diesen Verfahrensbedingungen cyclische Ketone aus cyclischen Alkenen mit 7 bis 20 C-Atomen hergestellt. WO 03/078371 offenbart ein Verfahren zur Herstellung substituierter Ketone aus substituierten Alkenen. WO 04/000777 offenbart ein Verfahren zur Umsetzung von Di- und Polyalkenen mit Distickstoffmonoxid zu den entsprechenden Carbonylverbindungen. Die Reinigung von Distickstoffmonoxid wird in diesen Schriften nicht erwähnt.

Es ist ebenfalls bekannt, dass Abgasströme enthaltend Distickstoffmonoxid für weitere Umsetzungen eingesetzt werden können. Distickstoffmonoxid fällt als unerwünschtes Nebenprodukt bei verschiedenen chemischen Prozessen an, insbesondere bei Oxidationen mit Salpetersäure und dort ganz besonders bei der Oxidation von Cyclohexanon und/oder Cyclohexanol zu Adipinsäure. Andere Beispiele für Verfahren, bei denen Distickstoffmonoxid als unerwünschtes Nebenprodukt anfällt, sind die Oxidation von Cyclododecanon und/oder Cyclododecanol mit Salpetersäure zu Dodecandicarbonsäure und die Partialoxidation von NH₃ zu NO.

So offenbaren die WO 2005/030690, die WO 2005/030689 und die WO 2004/096745 Verfahren zur Oxidation von Olefinen mit Distickstoffmonoxid, nämlich die Oxidation von Cyclododecatrien, von Cyclododecen und von Cyclopenten. Alle drei Anmeldungen offenbaren, dass neben anderen Distickstoffmonoxidquellen auch Abgasströme eingesetzt werden können, die beispielsweise durch destillative Methoden aufgereinigt werden können, bevor sie als Oxidationsmittel eingesetzt werden.

Sowohl bei der Herstellung von Distickstoffmonoxid als auch bei der Verwendung von Abgasströmen fällt N₂O zunächst als verdünntes gasförmiges Gemisch mit anderen Komponenten an. Diese Komponenten lassen sich unterteilen in solche, die für spezielle Anwendungen störend wirken, und solche, die sich inert verhalten. Für den Einsatz als Oxidationsmittel sind als solche störend wirkenden Gase unter anderem NOₓ oder beispielsweise Sauerstoff (O₂) zu nennen. Der Begriff "NOₓ", wie er im Rahmen der vorliegenden Erfindung verstanden wird, bezeichnet sämtliche Verbindungen NₐO_{b}, wobei a 1 oder 2 ist und b eine Zahl von 1 bis 6, außer N₂O. Statt dem Begriff "NOₓ" wird im Rahmen der vorliegenden Erfindung auch der Begriff "Stickoxide" verwendet. Als störende Nebenkomponenten sind auch NH₃ und organischen Säuren zu nennen.

Für spezielle Anwendungen ist es nötig, das eingesetzte Distickstoffmonoxid vor der Umsetzung zu reinigen. Beispielsweise für die Verwendung von Distickstoffmonoxid als Oxidationsmittel ist es nötig, störende Nebenkomponenten wie Sauerstoff oder Stickoxide NOₓ abzutrennen.

Verfahren zur Abtrennung von NOₓ sind grundsätzlich aus dem Stand der Technik bekannt. Eine Übersicht gibt beispielsweise M. Thiemann et. al in Ullmann's Encyclopedia, 6th Edition, 2000, Electronic Edition, Kapitel "Nitric Acid, Nitrous Acid, and Nitrogen Oxides", Abschnitt 1.4.2.3.

Die Anmeldung WO 00/73202 beschreibt eine Methode, wie man NOₓ und O₂ aus einem N₂O-haltigen Gasstrom entfernt werden kann. Das NOₓ wird durch katalytische Reduktion mit NH₃ entfernt und Sauerstoff durch katalytische Reduktion mit Wasserstoff oder anderen Reduktionsmitteln. Diese Methode hat aber den Nachteil, dass das Produkt mit NH₃ kontaminiert wird. Eine starke Abreicherung von Sauerstoff ist nur möglich, wenn ein Verlust an N₂O, von beispielsweise 3 bis 5% der ursprünglich enthaltenen Menge, in Kauf genommen wird.

Für spezielle Anwendungen kann es notwendig sein, auch die inerten Verbindungen abzutrennen, da sie die gewünschte Umsetzung mit N₂O durch Verdünnung verlangsamen können. Der Begriff "Inertgas", wie er im Rahmen der vorliegenden Erfindung verwendet wird, bezeichnet ein Gas, das sich hinsichtlich der Umsetzung von N₂O mit einem Olefin inert verhält, also unter den Bedingungen der Umsetzung von Olefinen mit N₂O weder mit den Olefinen noch mit N₂O reagieren. Als Inertgase sind beispielsweise Stickstoff, Kohlendioxid, Argon, Methan, Ethan und Propan zu nennen. Die l-nertgase können aber die Raum-Zeit-Ausbeute senken, so dass eine Abreicherung ebenfalls vorteilhaft sein kann. Es kann aber ebenfalls vorteilhaft sein, ein Gasgemisch zu erhalten, das noch Inertgase wie beispielsweise Kohlenstoffdioxid enthält, und das dann direkt in eine weitere Umsetzung eingesetzt werden kann.

In DE 27 32 267 A1 wird beispielsweise ein Verfahren zur Reinigung von Distickstoffmonoxid offenbart, wobei zunächst Stickstoffoxid, Stickstoffdioxid, Kohlenstoffdioxid und Wasser abgetrennt werden und anschließend das Gasgemisch durch Kompression auf 40 bis 300 bar und Kühlung auf 0 bis -88°C verflüssigt wird. Aus diesem verflüssigten Gasgemisch wird dann Distickstoffmonoxid abgetrennt. Diese Methode erreicht zwar eine Reinigung und Aufkonzentrierung des N₂O, ist aber aufgrund des geforderten hohen Drucks (60 bar), der tiefen Temperaturen (-85°C) und den damit verbundenen hohen Investitionen, wirtschaftlich unattraktiv.

In US 4,177,645 wird ein Verfahren zur Abtrennung von Distickstoffmonoxid aus Abgasströmen offenbart, das ebenfalls eine Vorreinigung und eine Tieftemperaturdestillation umfasst. Die Anmeldung EP 1 076 217 A1 beschreibt ebenfalls eine Methode zur Entfernung von leichtsiedenden Verunreinigungen aus N₂O durch Tieftemperaturdestillation.

Auch US 6,505,482, US 6,370,911 und US 6,387,161 offenbaren Verfahren zur Reinigung von Distickstoffmonoxid, bei dem jeweils eine Tieftemperaturdestillation in einer speziellen Anlage durchgeführt wird.

Eine Tieftemperaturdestillation erfordert durch die hohen Drücke und tiefen Temperaturen jedoch apparativ einen hohen Aufwand, der die Reinigung des Distickstoffmonoxids mit einem derartigen Verfahren aufwändig und kostenintensiv macht. Besonders störend ist hierbei die Tatsache, dass bei Normaldruck der Schmelzpunkt von N₂O nur 3K unterhalb des Siedepunkts liegt. Daher müssen hohe Drücke angewandt werden.

DE 20 40 219 offenbart ein Herstellungsverfahren für Distickstoffmonoxid, wobei das erhaltene Distickstoffmonoxid nach der Synthese konzentriert und gereinigt wird. Dabei wird gemäß der DE 20 40 219 zunächst Distickstoffmonoxid durch Oxidation von Ammoniak hergestellt. Das hergestellte Distickstoffmonoxid wird gereinigt, indem die oxidierten Gase separiert werden, und durch Absorption unter hohem Druck, der eine Desorption unter vermindertem Druck folgt, konzentriert. Nebenkomponenten werden beispielsweise durch Behandlung mit einer Alkalilösung in einem Waschturm entfernt. Als Lösungsmittel für die Absorption des Gasgemisches wird gemäß DE 20 40 219 Wasser verwendet.

Mit dem in DT 20 40 219 offenbarten Verfahren ist eine Trennung der verschiedenen Stickoxide möglich, das Verfahren erfordert jedoch den Einsatz von großen Lösungsmittelmengen und/oder hoher Drücke für die Absorption. Gleichzeitig ist für das gemäß DT 20 40 219 offenbarte Verfahren zur Abtrennung von weiteren störenden Komponenten ein weiterer Waschturm nötig.

In WO 2006/032502 wird ein Verfahren zur Reinigung eines Gasgemisches enthaltend Distickstoffmonoxid offenbart, das mindestens eine Absorption des Gasgemisches in einem organischen Lösungsmittel und anschließende Desorption des Gasgemisches aus dem beladenen organischen Lösungsmittel sowie das Einstellen des Gehalts an Stickoxiden NOₓ in dem Gasgemisch auf höchstens 0,5 Vol.-%, bezogen auf das Gesamtvolumen des Gasgemisches umfasst. In WO 2006/032502 wird auch offenbart, dass das Verfahren mehrere Absorptions- und Desorptionsschritte umfassen kann. In WO 2006/032502 werden nur organische Lösungsmittel als Absorptionsmedium offenbart.

Die DE 10 2005 055588.5 betrifft ein Verfahren zur Reinigung eines Gasgemisches G-0 enthaltend Distickstoffmonoxid, mindestens umfassend die Absorption des Gasgemisches G-0 in einem organischen Lösungsmittel, anschließende Desorption eines Gasgemisches G-1 aus dem beladenen organischen Lösungsmittel, Absorption des Gasgemisches G-1 in Wasser und anschließende Desorption eines Gasgemisches G-2 aus dem beladenen Wasser, sowie die Verwendung eines gereinigten Gasgemischs enthaltend Distickstoffmonoxid, erhältlich nach einem derartigen Verfahren als Oxidationsmittel für Olefine.

Die EP 06 125 807.5 betrifft ein Verfahren zur Reinigung eines Gasgemischs enthaltend Distickstoffmonoxid, wobei eine Absorption und Desorption in wässrigen Lösungsmittelgemischen bei bestimmten pH-Werten erfolgt.

Ausgehend von diesem Stand der Technik lag eine Aufgabe der vorliegenden Erfindung darin, ein Verfahren bereitzustellen, mit dem Distickstoffmonoxid-haltige Ströme effektiv und kostengünstig gereinigt und aufkonzentriert werden können. Derart aufgereinigtes Distickstoffmonoxid wird insbesondere als Oxidationsmittel benötigt.

Eine weitere Aufgabe der vorliegenden Erfindung war es, Verfahren zur Herstellung von Gasgemischen bereitzustellen, die ohne weitere Behandlung oder Zusatz anderer Inertisierungsmittel als Oxidationsmittel eingesetzt werden können und keine störenden Mengen an Verunreinigungen aufweist.

Erfindungsgemäß wird diese Aufgabe gelöst durch ein Verfahren zur Reinigung eines Gasgemischs enthaltend Distickstoffmonoxid mindestens umfassend die Schritte
(A) Behandeln eines Gasgemischs G-0 enthaltend Distickstoffmonoxid unter Erhalt eines Gasgemischs G-A mindestens umfassend die Schritte
   (i) Absorption des Gasgemischs G-0 in einem Lösungsmittelgemisch LM-I unter Erhalt eines Abgasstroms und einer Zusammensetzung Z-A
   (ii) Desorption eines Gasgemischs G-1 aus der Zusammensetzung Z-A unter Erhalt eines Lösungsmittelgemischs LM-I'
(B) Kondensation des Gasgemischs G-A unter Erhalt einer flüssigen Zusammensetzung Z-1 enthaltend Distickstoffmonoxid und einem gasförmigen Gemisch G-K,
wobei das gasförmige Gemisch G-K in die Behandlung gemäß Schritt (A) zurückgeführt wird.

Im Rahmen der vorliegenden Erfindung ist es auch möglich, das die Zusammensetzung des Gasgemischs G-A der des Gasgemischs G-1 entspricht.

Das erfindungsgemäße Verfahren hat unter anderem den Vorteil, dass neben den störenden Komponenten teilweise auch die inerten Komponenten abgetrennt werden. Das erfindungsgemäß gereinigte Distickstoffmonoxid wird also gleichzeitig aufkonzentriert. Durch das erfindungsgemäße Verfahren verbleibt jedoch Kohlenstoffdioxid, das inertisierend wirkt, zumindest teilweise in der flüssigen Zusammensetzung Z-1, so dass die flüssige Zusammensetzung Z-1 direkt sicherheitstechnisch unbedenklich als Oxidationsmittel ohne Zusatz weiterer Inertgase eingesetzt werden kann.

Somit ist es mit dem erfindungsgemäßen Verfahren möglich, ein sicherheitstechnisch unbedenkliches Oxidationsmittel als Gasgemisch bereitzustellen. Gleichzeitig wird durch die teilweise Kondensation und die Rückführung des gasförmigen Gemischs G-K die Ausbeute erhöht.

Durch die erfindungsgemäße Verfahrensführung können hohe Drücke und/oder sehr tiefe Temperaturen bei der Verflüssigung des Gasgemischs vermieden werden, da es durch die erfindungsgemäße Rückführung nicht notwendig ist, bei der Kondensation eine hohe Ausbeute zu erreichen.

Das erfindungsgemäße Verfahren umfasst einen Schritt (A), umfassend die Schritte (i) und (ii). Gemäß Schritt (A) wird ein Gasgemisch G-0 enthaltend Distickstoffmonoxid unter Erhalt eines Gasgemischs G-A behandelt, wobei der Schritt (A) mindestens die Schritte (i) und (ii) umfasst. Gemäß Schritt (i) wird das Gasgemisch G-0 in einem Lösungsmittelgemisch LM-I unter Erhalt eines Abgasstroms und einer Zusammensetzung Z-A absorbiert. Gemäß Schritt (ii) wird ein Gasgemisch G-1 aus der Zusammensetzung Z-A unter Erhalt eines Lösungsmittelgemischs LM-I' desorbiert.

Bei dem Gasgemisch G-0 handelt es sich im Rahmen der vorliegenden Erfindung um ein Gasgemisch enthaltend Distickstoffmonoxid, das gemäß dem erfindungsgemäßen Verfahren gereinigt wird. Dabei kann das Gasgemisch G-0 neben Distickstoffmonoxid weitere Komponenten enthalten.

Erfindungsgemäß kann das eingesetzte Gasgemisch G-0 enthaltend Distickstoffmonoxid grundsätzlich aus jeder beliebigen Quelle stammen.

Der Begriff "Gasgemisch", wie er im Rahmen der vorliegenden Erfindung verwendet wird, bezeichnet ein Gemisch aus zwei oder mehr Verbindungen, die sich bei Umgebungsdruck und Umgebungstemperatur im gasförmigen Zustand befinden. Bei veränderter Temperatur oder verändertem Druck kann das Gasgemisch auch in einem anderen Aggregatzustand vorliegen, beispielsweise flüssig, und wird im Rahmen der vorliegenden Erfindung weiter als Gasgemisch bezeichnet.

Im Rahmen der vorliegenden Erfindung wird die Zusammensetzung der Gasgemische oder der verflüssigten Gasgemische, wenn nicht ausdrücklich anders vermerkt, in Vol.-% angegeben. Dabei beziehen sich die Angaben auf die Zusammensetzung der Gasgemische bei Umgebungsdruck und Umgebungstemperatur.

Grundsätzlich kann die Zusammensetzung der Gemische im Rahmen der vorliegenden Erfindung auf jede dem Fachmann bekannte Weise bestimmt werden. Die Zusammensetzung der Gasgemische wird im Rahmen der vorliegenden Erfindung vorzugsweise gaschromatographisch bestimmt. Sie kann jedoch auch mittels UV-Spektroskopie, IR-Spektroskopie oder nasschemisch bestimmt werden.

Wird ein Gasgemisch G-0 eingesetzt, so ist dessen Gehalt an Distickstoffmonoxid im Wesentlichen beliebig, solange gewährleistet ist, dass die erfindungsgemäße Reinigung möglich ist.

Die N₂O-haltigen Gasgemische, die für dieses Verfahren als Gasgemisch G-0 eingesetzt werden, haben in der Regel einen N₂O-Gehalt zwischen 2 und 80 Vol.-% N₂O. Es enthält ferner beispielsweise 2 bis 21 Vol.-% O₂ und bis zu 30 Vol.-% NOₓ als unerwünschte Komponenten. Ferner kann es noch in wechselnden Mengen N₂, H₂, CO₂, CO, H₂O, NH₃ enthalten, in Spuren können auch noch organische Verbindungen enthalten sein. Beispielsweise kann das Gasgemisch G-0 auch 9 bis 13 Vol.-% N₂ und bis zu 5,5 Vol.-% NH₃ enthalten. Dabei ergibt die Summe der Komponenten des Gasgemischs G-0 100 Vol.-%.

Gemäß einer bevorzugten Ausführungsform des erfindungsgemäßen Verfahrens wird ein mindestens 3 Vol.-% Distickstoffmonoxid enthaltendes Gasgemisch G-0 eingesetzt, wobei wiederum bevorzugt Gemische mit einem Distickstoffmonoxid-Gehalt im Bereich von 4 bis 60 Vol.-%, weiter bevorzugt im Bereich von 5 bis 25 Vol.-% und insbesondere bevorzugt im Bereich von 6 bis 18 Vol.-% eingesetzt werden.

Das Gasgemisch G-0 weist gemäß dieser Ausführungsform vorzugsweise einen N₂O Gehalt von 6 bis 18 Vol.-%, besonders bevorzugt beispielsweise 7 Vol.-%, 8 Vol.-%, 9 Vol.-%, 10 Vol.-%, 11 Vol.-%, 12 Vol.-%, 13 Vol.-%, 14 Vol.-%, 15 Vol.-%, 16 Vol.-% oder 17 Vol.-% auf.

Das Gasgemisch G-0 hat beispielsweise einen Gehalt an CO₂ von 0,1 bis 7,5 Vol.-%, vorzugsweise von 0,5 bis 5 Vol.-%, besonders bevorzugt 1 bis 2,5 Vol.-%. Gleichzeitig hat das Gasgemisch G-0 beispielsweise einen Gehalt an O₂ von 1 bis 10 Vol.-%, vorzugsweise von 2 bis 7,5 Vol.-%, besonders bevorzugt beispielsweise 3,0 bis 6 Vol.-%. Darüber hinaus kann das Gasgemisch G-0 noch 50 bis 95 Vol.-% N₂ enthalten, vorzugsweise 60 bis 90 Vol.-%, besonders bevorzugt 70 bis 85 Vol.-%, sowie weitere Komponenten, beispielsweise Stickoxide oder Lösungsmittelreste. NOₓ kann dabei beispielsweise in einer Menge von 0 bis 0,2 Vol.-% enthalten sein, vorzugsweise 0,0001 bis 0,15 Vol.-%, besonders bevorzugt 0,0005 bis 0,1 Vol.-%. Dabei ergibt die Summe der Komponenten des Gasgemischs G-0 100 Vol.-%.

Gemäß einer bevorzugten Ausführungsform der vorliegenden Erfindung ist das Gasgemisch G-0 enthaltend Distickstoffmonoxid mindestens ein Distickstoffmonoxid enthaltendes Abgas eines chemischen Verfahrens. Im Rahmen der vorliegenden Erfindung sind auch Ausführungsformen umfasst, bei denen mindestens zwei Stickstoffmonoxid enthaltende Abgase einer einzigen Anlage als Gasgemisch enthaltend Distickstoffmonoxid dienen. Ebenso sind Ausführungsformen umfasst, bei denen mindestens ein Distickstoffmonoxid enthaltendes Abgas einer Anlage und mindestens ein weiteres Distickstoffmonoxid enthaltendes Abgas mindestens einer weiteren Anlage als Gasgemisch enthaltend Distickstoffmonoxid dienen.

Demgemäß betrifft die vorliegende Erfindung auch ein wie oben beschriebenes Verfahren, wobei das Gasgemisch enthaltend Distickstoffmonoxid mindestens ein Distickstoffmonoxid enthaltendes Abgas mindestens eines industriellen Verfahrens ist.

Der Begriff "Gasgemisch enthaltend Distickstoffmonoxid" bezeichnet im Rahmen der vorliegenden Erfindung sowohl Ausführungsformen, in denen das genannte Abgas in unmodifizierter Form dem erfindungsgemäßen Reinigungsverfahren unterworfen wird, als auch Ausführungsformen, in denen mindestens eines der genannten Abgase einer Modifikation unterworfen wird.

Der Begriff "Modifikation", wie er in diesem Zusammenhang im Rahmen der vorliegenden Erfindung verwendet wird, bezeichnet jedes geeignete Verfahren, mit dem die chemische Zusammensetzung eines Gasgemisches verändert wird. Demgemäß umfasst der Begriff "Modifikation" unter anderem Ausführungsformen, in denen ein Distickstoffmonoxid enthaltendes Abgas bezüglich des Distickstoffmonoxid-Gehaltes gemäß mindestens einem geeigneten Verfahrens aufkonzentriert wird. Vorzugsweise wird das Abgas keiner Modifikation unterworfen.

Gemäß einer weiteren Ausführungsform kann die chemische Zusammensetzung eines Abgases auch durch Zusatz von reinem Distickstoffmonoxid zu dem Abgas verändert werden.

Das eingesetzte Gasgemisch G-0 enthaltend N₂O kann beispielsweise ein Abgas aus einem industriellen Verfahren sein. Vorzugsweise stammt es aus einem Abgas einer Anlage zur Herstellung von Carbonsäuren durch Oxidation von Alkoholen, Aldehyden oder Ketonen mit Salpetersäure, wie z.B. aus einer Adipinsäure-, einer Dodecandicarbonsäure- oder einer Glyoxal-Anlage, aus dem Abgas einer Salpetersäure-Anlage die die obigen Abgasströme als Edukt einsetzt, aus dem Abgas einer Anlage zur Partialoxidation von NH₃ oder aus dem Abgas einer Anlage die die darin erzeugten Gasgemische einsetzt, wie z.B. einer Hydroxylamin-Anlage.

Erfindungsgemäß kann auch ein Gemisch verschiedener Abgase eingesetzt werden.

Gemäß einer weiter bevorzugten Ausführungsform der vorliegenden Erfindung stammt das mindestens eine Distickstoffmonoxid enthaltende Abgas aus einer Adipinsäureanlage, einer Dodecandicarbonsäureanlage, einer Glyoxal-Anlage, einer Hydroxylaminanlage und/oder einer Salpetersäureanlage, wobei letztere wiederum bevorzugt mit mindestens einem Abgas einer Adipinsäureanlage, einer Dodecandicarbonsäureanlage oder einer Glyoxal-Anlage betrieben wird.

Gemäß einer bevorzugten Ausführungsform wird der Abgasstrom einer Adipinsäureanlage eingesetzt, bei der durch Oxidation von Cyclohexanol/Cyclohexanon-Gemischen mit Salpetersäure pro Mol gebildeter Adipinsäure im Allgemeinen 0,8 bis 1,0 mol N₂O gebildet werden. Wie beispielsweise in A. K. Uriarte et al., Stud. Surf. Sci. Catal. 130 (2000) S. 743-748 beschrieben, enthalten die Abgase von Adipinsäureanlagen in unterschiedlichen Konzentrationen noch weitere Bestandteile wie unter anderem Stickstoff, Sauerstoff, Kohlendioxid, Kohlenmonoxid, Stickoxide, Wasser und flüchtige organische Verbindungen.

Bei der obenstehend erwähnten Dodecandicarbonsäureanlage handelt es sich um den im Wesentlichen identischen Anlagentyp.

Eine beispielsweise typische Zusammensetzung eines Abgases einer Adipinsäureanlage oder einer Dodecandicarbonsäureanlage ist in der folgenden Tabelle wiedergegeben:

| Komponente | Konzentrationen / Gew.-% |
|---|---|
| NO_{X} | 19 - 25 |
| N₂O | 20 - 28 |
| N₂ | 30 - 40 |
| O₂ | 7 - 10 |
| CO₂ | 2 - 3 |
| H₂O | ~ 7 |

Der Abgasstrom einer Adipinsäureanlage oder einer Dodecandicarbonsäureanlage kann direkt in dem erfindungsgemäßen Verfahren eingesetzt werden.

Gemäß einer ebenfalls bevorzugten Ausführungsform wird der Abgasstrom einer Salpetersäureanlage eingesetzt, die ganz oder teilweise mit Distickstoffmonoxid und Stickoxide enthaltenden Abgasen aus anderen Verfahren gespeist wird. In derartigen Salpetersäureanlagen werden Stickoxide adsorbiert und zum größten Teil zu Salpetersäure umgesetzt, während Distickstoffmonoxid nicht umgesetzt wird. Beispielsweise kann eine derartige Salpetersäureanlage durch Stickoxide, die durch gezielte Verbrennung von Ammoniak hergestellt werden, und durch Abgase einer Adipinsäureanlage und/oder durch Abgase einer Dodecandicarbonsäureanlage und/oder durch Abgase einer Glyoxal-Anlage gespeist werden. Ebenso ist es möglich, eine derartige Salpetersäureanlage allein durch Abgase einer Adipinsäureanlage und/oder durch Abgase einer Dodecandicarbonsäureanlage und/oder durch Abgase einer Glyoxal-Anlage zu speisen.

Die Abgase von derartigen Salpetersäureanlagen enthalten grundsätzlich in unterschiedlichen Konzentrationen noch weitere Bestandteile wie unter anderem Stickstoff, Sauerstoff, Kohlendioxid, Kohlenmonoxid, Stickoxide, Wasser und flüchtige organische Verbindungen.

Eine beispielsweise typische Zusammensetzung eines Abgases einer derartigen Salpetersäureanlage ist in der folgenden Tabelle wiedergegeben:

| Komponente | Konzentrationen / Gew.-% |
|---|---|
| NOₓ | < 0,1 |
| N₂O | 4 - 36 |
| N₂ | 57 - 86 |
| O₂ | 3 - 9 |
| CO₂ | 1 - 4 |
| H₂O | -0,6 |

Der Abgasstrom einer Salpetersäureanlage kann direkt in dem erfindungsgemäßen Verfahren eingesetzt werden.

Gemäß einer ebenfalls bevorzugten Ausführungsform des erfindungsgemäßen Verfahrens wird der Abgasstrom einer Hydroxylaminanlage eingesetzt, bei der beispielsweise zunächst Ammoniak mit Luft oder Sauerstoff zu NO oxidiert wird, wobei kleine Mengen an Distickstoffmonoxid als Nebenprodukt gebildet werden. Das NO wird anschließend mit Wasserstoff zu Hydroxylamin hydriert. Nachdem Distickstoffmonoxid unter den Hydrierbedingungen inert ist, reichert es sich im Wasserstoffkreis an. In bevorzugten Verfahrensführungen enthält der Purge-Strom einer Hydroxylaminanlage Distickstoffmonoxid im Bereich von 9 bis 13 Vol.-% in Wasserstoff. Dieser Purge-Strom kann als solcher zur erfindungsgemäßen Reinigung eingesetzt werden. Ebenso ist es möglich, diesen Strom bezüglich des Distickstoffmonoxid-Gehaltes, wie oben beschrieben, geeignet aufzukonzentrieren.

Demgemäß betrifft die vorliegende Erfindung auch ein wie oben beschriebenes Verfahren, wobei das Gasgemisch G-0 das Abgas einer Adipinsäureanlage und/oder einer Dodecandicarbonsäureanlage und/oder einer Glyoxal-Anlage und/oder einer Hydroxylaminanlage und/oder einer mit dem Abgas einer Adipinsäureanlage und/oder einer Dodecandicarbonsäureanlage und/oder einer Glyoxal-Anlage betriebenen Salpetersäureanlage ist.

Ebenso kann im Rahmen des erfindungsgemäßen Verfahrens Distickstoffmonoxid zum Einsatz im Verfahren gezielt hergestellt werden. Bevorzugt wird dabei unter anderem die Herstellung über die thermische Zersetzung von NH₄NO₃, wie dies beispielsweise in US 3,656,899 beschrieben ist. Ebenfalls bevorzugt wird weiter die Herstellung über die katalytische Oxidation von Ammoniak, wie dies beispielsweise in US 5,849,257 oder in WO 98/25698 beschrieben ist.

Bei der Absorption gemäß Schritt (i) wird das Gasgemisch G-0 in einem Lösungsmittelgemisch LM-I absorbiert. Dabei ist es im Rahmen der vorliegenden Erfindung grundsätzlich jede dem Fachmann bekannte Methode zur Absorption einzusetzen. Dabei wird ein Abgasstrom und eine Zusammensetzung Z-A erhalten. Die Zusammensetzung Z-A wird dann in Schritt (ii) weiter behandelt. Dabei wird das Gasgemisch G-1 aus der Zusammensetzung Z-A unter Erhalt eines Lösungsmittelgemischs LM-I' desorbiert.

Das Gasgemisch G-1 enthält dabei erfindungsgemäß mindestens Distickstoffmonoxid und kann weitere Komponenten enthalten.

Erfindungsgemäß kann als Lösungsmittelgemisch LM-I jedes dem Fachmann bekannte geeignete Lösungsmittelgemisch eingesetzt werden, solange gewährleistet ist, dass das Gasgemisch G-0, insbesondere Distickstoffmonoxid, zumindest teilweise absorbiert wird.

Gemäß Schritt (A) wird ein Gasgemisch G-A erhalten, das Distickstoffmonoxid enthält. Das Gasgemisch G-A kann darüber hinaus weitere Komponenten enthalten. Sofern der Schritt (A) nach dem Schritt (ii) keine weiteren Schritte umfasst, ist die Zusammensetzung des Gasgemischs G-1 identisch mit der des Gasgemischs G-A.

Gemäß Schritt (B) wird das aus Schritt (A) erhaltene Gasgemisch G-A unter Erhalt einer flüssigen Zusammensetzung Z-1 enthaltend Distickstoffmonoxid und einem gasförmigen Gemisch G-K zumindest teilweise kondensiert. Dabei enthält im Rahmen der vorliegenden Erfindung die flüssige Zusammensetzung Z-A Distickstoffmonoxid und kann weitere Komponenten enthalten. Das gasförmige Gemisch G-K enthält im Rahmen der vorliegenden Erfindung vorzugsweise nur geringe Mengen an Distickstoffmonoxid. Gemäß dem erfindungsgemäßen Verfahren wird nach der Kondensation gemäß Schritt (B) das gasförmige Gemisch G-K in die Behandlung gemäß Schritt (A) zurückgeführt.

Somit ist es gemäß dem erfindungsgemäßen Verfahren möglich, auch geringe Mengen Distickstoffmonoxid, die in dem Gemisch G-K enthalten sein können, in das Verfahren zurückzuführen, um die Ausbeute zu erhöhen.

Erfindungsgemäß kann das Verfahren weitere Schritte umfassen. So ist es beispielsweise im Rahmen der vorliegenden Erfindung möglich, dass zwischen den Schritten (A) und (B) weitere Schritte umfasst sind.

Erfindungsgemäß kann der Schritt (A) auch weitere Schritte umfassen. Dabei ist es insbesondere möglich, dass der Schritt (A) eine weitere Absorption des Gasgemischs G-1 in einem geeigneten Lösungsmittelgemisch und eine weitere Desorption umfasst.

Daher betrifft die vorliegende Erfindung gemäß einer weiteren Ausführungsform ein wie zuvor beschriebenes Verfahren Reinigung eines Gasgemischs enthaltend Distickstoffmonoxid, wobei der Schritt (A) zusätzlich die Schritte (iii) und (iv) umfasst:
(iii) Absorption des Gasgemischs G-1 in einem Lösungsmittelgemisch LM-II unter Erhalt eines Abgasstroms und einer Zusammensetzung Z-B
(iv) Desorption eines Gasgemischs G-2 aus der Zusammensetzung Z-B unter Erhalt eines Lösungsmittelgemischs LM-II'.

Erfindungsgemäß kann als Lösungsmittelgemisch LM-II jedes dem Fachmann bekannte geeignete Lösungsmittelgemisch eingesetzt werden, solange gewährleistet ist, dass das Gasgemisch G-1, insbesondere Distickstoffmonoxid, zumindest teilweise absorbiert wird.

Sofern der Schritt (A) nach dem Schritt (iv) keine weiteren Schritte umfasst, ist die Zusammensetzung des Gasgemischs G-2 identisch mit der des Gasgemischs G-A.

Im Rahmen der vorliegenden Erfindung ist es auch möglich, dass der Schritt (A) neben den Schritten (i) und (ii) oder neben den Schritten (i), (ii), (iii) und (iv) weitere Schritte umfasst, auch weitere Absorptionen und Desorptionen.

So ist es im Rahmen der vorliegenden Erfindung möglich, dass das Verfahren mehrere Schritte (i) und (ii) oder mehrere Schritte (iii) und (iv) umfasst.

Daher betrifft die vorliegende Erfindung gemäß einer weiteren Ausführungsform ein wie zuvor beschriebenes Verfahren zur Reinigung eines Gasgemischs enthaltend Distickstoffmonoxid, wobei der Schritt (A) weitere Schritte umfasst.

Wie zuvor beschrieben, wird das gemäß Schritt (B) erhaltene gasförmige Gemisch G-K in den Schritt (A) des erfindungsgemäßen Verfahrens zurückgeführt. Dabei wird im Rahmen der vorliegenden Erfindung das gasförmige Gemisch G-K mit einem anderen Gasgemisch gemischt. Vorzugsweise wird dabei das gasförmige Gemisch G-K derart in den Schritt (A) zurückgeführt, dass eine Gewinnung des gegebenenfalls im gasförmige Gemisch G-K enthaltenen Distickstoffmonoxids möglich ist. Daher ist es im Rahmen der vorliegenden Erfindung bevorzugt, dass das gasförmige Gemisch G-K mit einem Gasgemisch gemischt wird, das einer Absorption zugeführt wird, insbesondere dem Gasgemisch G-0 oder dem Gasgemisch G-1. Somit ist es im Rahmen der vorliegenden Erfindung bevorzugt, das gasförmige Gemisch G-K in Schritt (i) oder in Schritt (iii) des Schritts (A) zurückzuführen.

Daher betrifft die vorliegende Erfindung gemäß einer weiteren Ausführungsform ein wie zuvor beschriebenes Verfahren zur Reinigung eines Gasgemischs enthaltend Distickstoffmonoxid, wobei das gasförmige Gemisch G-K in Schritt (i) oder in Schritt (iii) des Schritts (A) zurückgeführt wird.

Das erfindungsgemäße Verfahren umfasst gemäß Schritt (A) mindestens die Schritte (i) und (ii) und gemäß einer weiteren Ausführungsform auch die Schritte (iii) und (iv), wobei die Lösungsmittelgemische LM-I und LM-II eingesetzt werden.

Erfindungsgemäß kann als Lösungsmittelgemisch LM-I und oder LM-II jedes dem Fachmann bekannte geeignete Lösungsmittelgemisch eingesetzt werden, solange gewährleistet ist, dass insbesondere Distickstoffmonoxid absorbiert wird.

Geeignete Lösungsmittelgemische LM-I und LM-II für die Absorption gemäß Schritt (i) oder (iii) sind solche, die für N₂O und vorzugsweise auch CO₂ als inerter Komponente eine bessere Löslichkeit aufweisen, als für die unerwünschten Komponenten des eintretenden Eduktgases G-0.

Erfindungsgemäß können als Lösungsmittelgemisch LM-I und/oder LM-II organische Lösungsmittel oder wässrige Lösungsmittelgemische eingesetzt werden. Daher betrifft die vorliegende Erfindung gemäß einer weiteren Ausführungsform ein wie zuvor beschriebenes Verfahren zur Reinigung eines Gasgemischs enthaltend Distickstoffmonoxid, wobei das Lösungsmittelgemisch LM-I oder das Lösungsmittelgemisch LM-II oder das Lösungsmittelgemisch LM-I und das Lösungsmittelgemisch LM-II ausgewählt ist aus der Gruppe bestehend aus organischen Lösungsmitteln und wässrigen Lösungsmittelgemischen.

Als organische Lösungsmittel können erfindungsgemäß alle Lösungsmittel eingesetzt werden, bei denen das Verhältnis zwischen N₂O-Löslichkeit (in mol/mol Lösungsmittel) und der Löslichkeit der unerwünschten Nebenkomponenten unter den im Absorber herrschenden Bedingungen (dieses Verhältnis wird im folgenden γ genannt) mindestens 5 beträgt. Dies Verhältnis kann für jede einzelne im Gasgemisch enthaltene Komponente bestimmt werden. Bevorzugte organische Lösungsmittel weisen beispielsweise bei 30 °C einen Wert γ_{O2} von 6 bis 30, bevorzugt von 9 bis 25 und einen Wert y_{N2} von größer 10, bevorzugt von größer als 15, insbesondere von größer als 20 auf.

Beispiele für geeignete organische Lösungsmittel sind beispielsweise aliphatische Kohlenwasserstoffe, bevorzugt mit mindestens 5 C-Atomen, weiter bevorzugt mit mindestens 8 C-Atomen, substituierte oder unsubstituierte aromatische Kohlenwasserstoffe, Ester, Ether, Amide, Lactone, Lactame, Nitrile, Alkylhalogenide, Olefine oder Mischungen dieser Lösungsmittel.

Ganz besonders bevorzugt sind erfindungsgemäß organische Lösungsmittel, die einen Siedepunkt bei Normaldruck von mindestens 100°C aufweisen, da dadurch die Lösungsmittelverluste sowohl im Abgasstrom des Absorbers wie auch des Desorbers reduziert werden.

Darüber hinaus weisen erfindungsgemäß geeignete Lösungsmittel gleichzeitig eine gute Löslichkeit für Distickstoffmonoxid auf. Die Löslichkeit wird über das Verhältnis zwischen dem Partialdruck von N₂O in der Gasphase und dem Molanteil von N₂O in der Flüssigphase (Henry-Koeffizient, H_{N2O}) angegeben, d.h. ein kleiner Wert bedeutet eine hohe Löslichkeit von Distickstoffmonoxid im Lösungsmittel. Vorzugsweise ist dies Verhältnis für ein insbesondere in dem ersten Schritt eingesetztes organisches Lösungsmittel bei 30°C kleiner als 1000, weiter bevorzugt kleiner als 750, besonders bevorzugt kleiner als 500, insbesondere kleiner als 150.

Geeignete organische Lösungsmittel sind unter anderem N-Methylpyrrolidon, Dimethylformamid, Dimethylsulfoxid, Propylencarbonat, Sulfolan, N,N-Dimethylacetamid oder Cyclopentan. Besonders bevorzugt sind im Rahmen der vorliegenden Erfindung beispielsweise Toluol, Nitrobenzol, 1,2-Dichlorbenzol, Tetradecan, beispielsweise ein technisches Gemisch aus gesättigten Kohlenwasserstoffen mit überwiegend 14 Kohlenstoffatomen, und Phthalsäuredimethylester.

Daher betrifft die vorliegende Erfindung in einer bevorzugten Ausführungsform ein Verfahren zur Reinigung eines Gasgemisches umfassend Distickstoffmonoxid wie oben beschrieben, wobei das organische Lösungsmittel ausgewählt ist aus der Gruppe bestehend aus Toluol, Nitrobenzol, 1,2-Dichlorbenzol, Tetradecan und Phthalsäuredimethylester.

Erfindungsgemäß ist es ebenfalls möglich, als Lösungsmittelgemisch LM-I und/oder LM-II wässrige Lösungsmittelgemische einzusetzen. Dabei gelten für die Eignung der Lösungsmittelgemische für das erfindungsgemäße Verfahren grundsätzlich die obigen Ausführungen. Insbesondere können als Lösungsmittelgemisch LM-I und/oder LM-II Lösungsmittelgemische mindestens enthaltend 50 Gew.-% Wasser bezogen auf das gesamte Lösungsmittelgemisch, eingesetzt werden. Dabei ist es im Rahmen der vorliegenden Erfindung auch möglich, dass der pH-Wert des eingestzten Lösungsmittelgemischs in einem bestimmten Bereich eingestellt wird. Ein geeigneter pH-Wert für ein wässriges Lösungsmittelgemisch liegt erfindungsgemäß beispielsweise im Bereich von 2,0 bis 8,0. Dabei ist es erfindungsgemäß, auch möglich, dass der pH-Wert der in den einzelnen Absorptionsschritten eingesetzten Lösungsmittelgemische LM-I oder LM-II variiert.

Im Kontext dieser Anmeldung wird der pH-Wert mit einer kommerziell erhältlichen Glaselektrode gemessen, die vorher gegen Puffer mit bekanntem pH-Wert kalibriert wurde. Alle Angaben von pH-Werten beziehen sich auf eine Messung mit einer kalibrierten und temperaturkompensierten Glaselektrode. Falls die Kalibriertemperatur von der Messtemperatur abweicht wird eine Temperaturkompensierung verwendet. Diese Definition und dieses Vorgehen entspricht der derzeit gültigen IUPAC Empfehlung (R.P.Buck et al., Pure Appl. Chem. (2002) 74(11), S. 2169-2200 und dort insbesondere Abschnitt 11).

Wasser weist eine hohe Selektivität für die gewünschten Komponenten, insbesondere Distickstoffmonoxid und Kohlenstoffdioxid, auf. Gleichzeitig ist die absolute Löslichkeit von Distickstoffmonoxid in Wasser ausreichend, um eine weitere Aufkonzentrierung zu erreichen. Dabei hat Wasser als Lösungsmittel den Vorteil, dass auch unter Druck in Gegenwart von konzentriertem Distickstoffmonoxid keine sicherheitstechnischen Probleme auftreten. Gleichzeitig kann keine Kontaminierung des Gasgemischs G-2 mit einem organischen Lösungsmittel auftreten, die zusätzliche Reinigungsschritte nötig machen würden.

Erfindungsgemäß können sowohl das Lösungsmittelgemisch LM-I als auch LM-II ein organisches Lösungsmittelgemisch oder ein wässriges Lösungsmittelgemisch sein. Erfindungsgemäß ist es möglich, dass als Lösungsmittelgemisch LM-I ein organisches Lösungsmittel eingesetzt wird und als Lösungsmittelgemisch LM-II ein wässriges Lösungsmittelgemisch. Ebenso ist es möglich, dass als Lösungsmittelgemisch LM-I ein wässriges Lösungsmittelgemisch eingesetzt wird und als Lösungsmittelgemisch LM-II ein organisches Lösungsmittel. Vorzugsweise sind im Rahmen der vorliegenden Erfindung sowohl das Lösungsmittelgemisch LM-I als auch das Lösungsmittelgemisch LM-II ein wässriges Lösungsmittelgemisch.

Weiter ist es bevorzugt, dass sofern als Lösungsmittelgemisch LM-I und/oder LM-II ein wässriges Lösungsmittelgemisch eingesetzt wird, der pH-Wert des wässrigen Lösungsmittelgemischs in einem bestimmten Bereich eingestellt wird.

Durch die erfindungsgemäße Auswahl des pH-Wertes von Lösungsmittelgemisch LM-I und Lösungsmittelgemisch LM-II wird eine fast vollständige Abreicherung von NOₓ erzielt. Dadurch wird eine separate Abtrennung von NOₓ, beispielsweise mittels DeNOx oder SCR-DeNOx überflüssig. Dadurch gibt es bei dem erfindungsgemäßen Verfahren beispielsweise auch keine Gefahr der Kontamination vom Produktstrom mit NH₃ der für die DeNOx-Stufe als Reduktionsmittel verwendet wird.

Durch die erfindungsgemäß bevorzugte gezielte Auswahl des pH-Wertes des Lösungsmittelgemischs LM-I und des Lösungsmittelgemischs LM-II kann insbesondere eine gute Abreicherung von NOₓ mit nur einer minimalen Änderung des Kohlenstoffdioxid-Gehaltes erreicht werden.

Das erfindungsgemäß eingesetzte Lösungsmittelgemisch LM-I und LM-II weisen bei dem erfindungsgemäß bevorzugten pH-Wert eine hohe Selektivität für die gewünschten Komponenten, insbesondere Distickstoffmonoxid und Kohlenstoffdioxid, auf. Gleichzeitig ist die absolute Löslichkeit von Distickstoffmonoxid in dem erfindungsgemäß eingesetzten Lösungsmittelgemisch LM-I bzw. LM-II ausreichend, um eine Aufkonzentrierung zu erreichen. Dabei hat das erfindungsgemäß eingesetzte Lösungsmittelgemisch LM-I bzw. LM-II den Vorteil, dass auch unter Druck in Gegenwart von konzentriertem Distickstoffmonoxid keine sicherheitstechnischen Probleme auftreten.

Erfindungsgemäß kann der pH-Wert des wässrigen Lösungsmittelgemischs bei der Absorption vorzugsweise im Bereich von 3,5 bis 8,0 liegen. Bei diesem pH-Wert erfolgt erfindungsgemäß eine gute Absorption von Distickstoffmonoxid und Kohlenstoffdioxid im Lösungsmittelgemisch, wobei andere Gase, die im Gasgemisch G-0 enthalten sein können, nicht oder wenig absorbiert werden. Bevorzugt liegt der pH-Wert in einem Bereich von 5,0 bis 7,5 besonders bevorzugt in einem Bereich von 6,0 bis 7,0.

Dabei wird erfindungsgemäß der pH-Wert vor oder während des Inkontaktbringens des Gasgemischs mit dem wässrigen Lösungsmittelgemisch gemessen und dann beispielsweise der pH-Wert durch geeignete Maßnahmen eingestellt. Ebenso ist es erfindungsgemäß möglich, dass keine Maßnahmen nötig sind, um den pH-Wert einzustellen.

Grundsätzlich kann der pH-Wert erfindungsgemäß durch alle dem Fachmann bekannten Maßnahmen eingestellt werden. Geeignete Maßnahmen zum Einstellen des pH-Werts sind beispielsweise Zugabe einer Säure oder Lauge oder Zugabe von weiterem Lösungsmittel.

Beispielsweise wird der pH-Wert des wässrigen Lösungsmittelgemischs vor oder nach der Absorption gemessen und der pH-Wert im erfindungsgemäßen Bereich durch geeignete Maßnahmen eingestellt. Die Messung des pH-Werts kann dabei erfindungsgemäß kontinuierlich oder diskontinuierlich erfolgen.

Sofern der pH-Wert des Lösungsmittelgemischs LM-I und des Lösungsmittelgemischs LM-II eingestellt werden, können der pH-Wert des Lösungsmittelgemischs LM-I und des Lösungsmittelgemischs LM-II unabhängig voneinander eingestellt werden. Erfindungsgemäß ist es auch möglich, dass nur der pH-Wert des Lösungsmittelgemischs LM-I oder des Lösungsmittelgemischs LM-II eingestellt werden. Der pH-Wert des Lösungsmittelgemischs LM-I und des Lösungsmittelgemischs LM-II können jedoch erfindungsgemäß auch im gleichen Bereich eingestellt werden.

Im Rahmen der vorliegenden Erfindung wird unter einem wässrigen Lösungsmittelgemisch ein Lösungsmittelgemisch verstanden, mindestens enthaltend 50 Gew.-% Wasser beispielsweise 50 bis 100 Gew.-% Wasser, vorzugsweise mindestens 60 Gew.-% Wasser, insbesondere mindestens 70 Gew.-% Wasser, besonders bevorzugt mindestens 80 Gew.-% Wasser, beispielsweise mindestens 90 Gew.-% Wasser. Vorzugsweise enthält das wässrige Lösungsmittelgemisch mindestens 90 Gew.-% Wasser, jeweils bezogen auf das gesamte wässrigen Lösungsmittelgemisch.

Daher betrifft die vorliegende Erfindung auch ein wie zuvor beschriebenes Verfahren zur Reinigung eines Gasgemischs G-0 enthaltend Distickstoffmonoxid, wobei das Lösungsmittelgemisch LM-I oder das Lösungsmittelgemisch LM-II oder das Lösungsmittelgemisch LM-I und das Lösungsmittelgemisch LM-II mindestens 90 Gew.-% Wasser enthält, jeweils bezogen auf das gesamte Lösungsmittelgemisch.

Erfindungsgemäß kann das wässrige Lösungsmittelgemisch neben Wasser auch andere polare mit Wasser mischbare Lösungsmittel enthalten, beispielsweise Glykole. Darüber hinaus kann das wässrigen Lösungsmittelgemisch neben Wasser auch gelöste Salze enthalten, beispielsweise Salze der Alkali- oder Erdalkalimetalle, insbesondere Hydroxide, Hydrogencarbonate, Carbonate, Nitrate, Nitrite, Sulfate, Hydrogenphosphate oder Phosphate.

Erfindungsgemäß ist der Gehalt an Salzen im wässrigen Lösungsmittelgemisch kleiner als 5 Gew.-%, bevorzugt kleiner als 2,5 Gew.-%, besonders bevorzugt kleiner als 2,0 Gew.-%. Der Gehalt an Salzen im wässrigen Lösungsmittelgemisch beträgt beispielsweise 0,0001 bis 5 Gew.-%, bevorzugt 0,001 bis 2,5 Gew.-%, insbesondere 0,01 bis 2,0 Gew.-%.

Erfindungsgemäß wird der Gehalt an Salzen im wässrigen Lösungsmittelgemisch vorzugsweise dadurch kontrolliert, dass kontinuierlich oder diskontinuierlich einen Teil des mit Salzen beladenen Lösungsmittelgemischs durch eine entsprechend angepasste Menge an frischem Lösungsmittelgemisch ersetzt wird.

Erfindungsgemäß kann der pH-Wert des wässrigen Lösungsmittelgemischs mittels jeder dem Fachmann bekannten Methode eingestellt werden. Insbesondere kann der pH-Wert durch Zugabe einer Base zu dem wässrigen Lösungsmittelgemisch eingestellt werden.

Prinzipiell kann als Base jede erdenkliche Verbindung eingesetzt werden, deren pH-Wert als 1 Gew.-%ige Lösung in Wasser >8,0 ist. Bevorzugt werden erfindungsgemäß starke anorganische Basen, insbesondere Hydroxide, Carbonate, Hydrogencarbonate oder Phosphate von Alkali- oder Erdalkalimetallen eingesetzt. Besonders bevorzugt werden NaOH, KOH, Na₂CO₃, NaHCO₃, Na₃PO₄, K₃PO₄ eingesetzt. Weiter bevorzugt ist der Einsatz der Basen in Form einer konzentrierten wässrigen Lösung.

Geeignete Konzentrationsbereiche sind im Rahmen der vorliegenden Erfindung beispielsweise 10 bis 60 %-ige wässrige Lösungen, bevorzugt 20 bis 55 %-ige wässrige Lösungen, besonders bevorzugt 25 bis 50 %-ige wässrige Lösungen, beispielsweise 30 %-ige wässrige Lösungen, 35 %-ige wässrige Lösungen, 40 %-ige wässrige Lösungen, 45 %-ige wässrige Lösungen oder 50 %-ige wässrige Lösungen.

Erfindungsgemäß besonders bevorzugt ist der Einsatz einer wässrige NaOH-Lösung als Base.

Gemäß einer bevorzugten Ausführungsform der vorliegenden Erfindung wird als Base eine 25 bis 50 %-ige wässrige NaOH-Lösung eingesetzt.

Beispielsweise wird der pH-Wert des wässrigen Lösungsmittelgemischs durch Zugabe einer Base ausgewählt aus der Gruppe bestehend aus Alkalimetallhydroxiden, Alkalimetallcarbonaten, Alkalimetallhydrogencarbonaten, Alkalimetallphosphaten, Erdalkalimetallhydroxiden, Erdalkalimetallcarbonaten, Erdalkalimetallhydrogencarbonaten und Erdalkalimetallphosphaten eingestellt.

Gemäß Schritt (i) erfolgt erfindungsgemäß eine zumindest teilweise Absorption des Gasgemischs G-0 in einem Lösungsmittelgemisch LM-I, wobei eine Zusammensetzung Z-A und ein an den absorbierten Gasen verarmter Abgasstrom erhalten wird.

Im Rahmen der vorliegenden Erfindung wird unter einem verarmten Abgasstrom ein Gasstrom verstanden, der die nicht bei der Absorption im Lösungsmittelgemisch LM-I oder LM-II absorbierten Gase enthält.

Die Zusammensetzung Z-A umfasst das Lösungsmittelgemisch LM-I und die darin absorbierten Gase.

Sofern als Lösungsmittelgemisch LM-I Wasser eingesetzt wird, enthält die Zusammensetzung Z-A beispielsweise 90,0 bis 99,9999 Gew.-% Wasser, insbesondere 95,0 bis 99,999 Gew.-%, bevorzugt 98,0 bis 99,99 Gew.-% Wasser; beispielsweise 0,01 bis 0,25 Gew.-% Distickstoffmonoxid, insbesondere 0,05 bis 0,2 Gew.-%, bevorzugt 0,1 bis 0,15 Gew.-% Distickstoffmonoxid; beispielsweise 0,0001 bis 0,1 Gew.-% Kohlenstoffdioxid, insbesondere 0,001 bis 0,05 Gew.-% Kohlenstoffdioxid; beispielsweise 0,0001 bis 0,1 Gew.-% Stickstoff, insbesondere 0,001 bis 0,05 Gew.-% Stickstoff; beispielsweise 0,05 bis 1,5 Gew.-% Natriumnitrit, insbesondere 0,1 bis 1,0 Gew.-%, bevorzugt 0,25 bis 0,75 Gew.-% Natriumnitrit; beispielsweise 0,05 bis 1,5 Gew.-% Natriumnitrat, insbesondere 0,1 bis 1,0 Gew.-%, bevorzugt 0,25 bis 0,75 Gew.-% Natriumnitrat; beispielsweise 0,0001 bis 0,1 Gew.-% Natriumhydrogencarbonat, insbesondere 0,001 bis 0,05 Gew.-% Natriumhydrogencarbonat; sowie Spuren von Sauerstoff und Argon. Dabei ergibt die Summe der Komponenten der Zusammensetzung Z-A 100 Gew.-%.

Erfindungsgemäß enthält der verarmte Abgasstrom beispielsweise 0,1 bis 2,0 Vol.-% Argon, insbesondere 0,25 bis 1,5 Vol.-%, bevorzugt 0,5 bis 1,0 Vol.-% Argon; beispielsweise 1,0 bis 10 Vol.-% Sauerstoff, insbesondere 2,5 bis 7,5 Vol.-%, bevorzugt 4,0 bis 6,0 Vol.-% Sauerstoff; beispielsweise 1,0 bis 10 Vol.-% Distickstoffmonoxid, insbesondere 2,5 bis 7,5 Vol.-%, bevorzugt 4,0 bis 6,0 Vol.-% Distickstoffmonoxid; beispielsweise 70 bis 99,9 Vol.-% Stickstoff, insbesondere 75 bis 95 Vol.-%, bevorzugt 80 bis 90 Vol.-% Stickstoff; beispielsweise 0,01 bis 0,5 Vol.-% Kohlenstoffmonoxid, insbesondere 0,05 bis 0,25 Vol.-%, bevorzugt 0,08 bis 0,1 Vol.-% Kohlenstoffmonoxid; beispielsweise 0,1 bis 1,5 Vol.-% Kohlenstoffdioxid, insbesondere 0,25 bis 1,0 Vol.-%, bevorzugt 0,5 bis 0,75 Vol.-% Kohlenstoffdioxid; beispielsweise 0,1 bis 1,5 Vol.-% Wasser, insbesondere 0,25 bis 1,0 Vol.-%, bevorzugt 0,5 bis 0,75 Vol.-% Wasser. Dabei ergibt die Summe der Komponenten des Abgasstroms 100 Vol.-%.

Vorzugsweise wird Schritt (i) des erfindungsgemäßen Verfahrens kontinuierlich durchgeführt. Das heißt im Rahmen der vorliegenden Erfindung, dass kontinuierlich das Lösungsmittelgemisch LM-I und das Gasgemisch G-0 in Kontakt gebracht werden, wobei sich kontinuierlich die Zusammensetzung Z-A und der verarmte Abgasstrom bilden.

Erfindungsgemäß werden bei der Absorption gemäß Schritt (i) vorzugsweise Distickstoffmonoxid und Kohlenstoffdioxid absorbiert. Erfindungsgemäß können beispielsweise auch Stickstoff, Sauerstoff und Argon absorbiert werden. Auch Stickoxide NOₓ werden gemäß Schritt (i) absorbiert.

Das erfindungsgemäße Verfahren umfasst weiter einen Schritt (ii), bei dem ein Gasgemisch G-1 aus der Zusammensetzung Z-A unter Erhalt eines Lösungsmittelgemischs LM-I' desorbiert wird.

Dabei werden gemäß Schritt (ii) vorzugsweise Distickstoffmonoxid und Kohlenstoffdioxid aus der Zusammensetzung Z-A desorbiert.

Das Lösungsmittelgemisch LM-I' enthält neben dem eingesetzten Lösungsmittelgemisch LM-I noch nicht desorbierte Gase und Folgeprodukte.

Beispielsweise für den Fall, dass im Erfindungsgemäßen Verfahren als Lösungsmittelgemisch LM-I mit einem bestimmten eingestellten pH-Wert eingesetzt wird und der pH-Wert durch Zugabe einer Lauge, insbesondere Natronlauge, eingestellt wird, enthält das Lösungsmittelgemisch LM-I' erfindungsgemäß beispielsweise 90,0 bis 99,9999 Gew.-% Wasser, insbesondere 95,0 bis 99,999 Gew.-%, bevorzugt 98,0 bis 99,99 Gew.-% Wasser; beispielsweise 0,001 bis 0,1 Gew.-% Distickstoffmonoxid, beispielsweise 0,05 bis 1,5 Gew.-% Natriumnitrit, insbesondere 0,1 bis 1,0 Gew.-%, bevorzugt 0,25 bis 0,75 Gew.-% Natriumnitrit; beispielsweise 0,05 bis 1,5 Gew.-% Natriumnitrat, insbesondere 0,1 bis 1,0 Gew.-%, bevorzugt 0,25 bis 0,75 Gew.-% Natriumnitrat; beispielsweise 0,0001 bis 0,1 Gew.-% Natriumhydrogencarbonat, insbesondere 0,001 bis 0,05 Gew.-% Natriumhydrogencarbonat. Das Lösungsmittelgemisch LM-I' kann darüber hinaus auch weitere Verbindungen enthalten. Dabei ergibt die Summe der Komponenten des Lösungsmittelgemischs LM-I' 100 Gew.-%.

Im Rahmen der vorliegenden Erfindung hat das Gasgemisch G-1 beispielsweise einen Gehalt an N₂O von 40 bis 80 Vol.-%, vorzugsweise von 45 bis 75 Vol.-%, insbesondere von 50 bis 65 Vol.-%, besonders bevorzugt beispielsweise 51 Vol.-%, 52 Vol.-%, 53 Vol.-% 54 Vol.-%, 55 Vol.-%, 56 Vol.-%, 57 Vol.-%, 58 Vol.-%, 59 Vol.-%, 60 Vol.-%, 61 Vol.-%, 62 Vol.-%, 63 Vol.-%, 64 Vol.-% oder 65 Vol.-%.

Das Gasgemisch G-1 hat beispielsweise einen Gehalt an CO₂ von 5 bis 15 Vol.-%, vorzugsweise von 6 bis 12 Vol.-%, besonders bevorzugt beispielsweise 7 Vol.-%, 9 Vol.-%, 10 Vol.-% oder 11 Vol.-%. Gleichzeitig hat das Gasgemisch G-1 beispielsweise einen Gehalt an O₂ von 1,0 bis 4,0 Vol.-%, vorzugsweise von 1,5 bis 3,5 Vol.-%, besonders bevorzugt 2,5 bis 3.1 Vol.-%, beispielsweise 2,6 Vol.-%, 2,7 Vol.-%, 2,8 Vol.-%, 2,9 Vol.-% oder 3,0 Vol.-%. Darüber hinaus kann das Gasgemisch G-1 noch 20 bis 40 Vol.-% N₂ enthalten, vorzugsweise 20 bis 35 Vol.-%, sowie weitere Komponenten, beispielsweise Stickoxide. NOₓ kann dabei beispielsweise in einer Menge von 0 bis 0,1 Vol.-% enthalten sein, vorzugsweise 0,0001 bis 0,01 Vol.-%, besonders bevorzugt 0,0002 bis 0,05 Vol.-%. Dabei ergibt die Summe der Komponenten des Gasgemischs G-1 100 Vol.-%. Das Gasgemisch G-1 kann darüber hinaus noch 0 bis 10 Vol.-% Wasser enthalten, insbesondere 2 bis 8 Vol.-%, bevorzugt 4 bis 6 Vol.-% Wasser.

Erfindungsgemäß kann der Schritt (A) weitere Schritte umfassen, insbesondere eine weitere Absorption und Desorption in einem geeigneten Lösungsmittel gemäß den Schritten (iii) und (iv). Gemäß Schritt (iii) und (iv) erfolgt eine Absorption des Gasgemischs G-1 in einem geeigneten Lösungsmittelgemisch LM-II und eine anschließende Desorption des Gasgemischs G-2.

Das erfindungsgemäße Verfahren kann weiter einen Schritt (iii) und einen Schritt (iv) aufweisen.

Bei der Absorption gemäß Schritt (iii) erfolgt erfindungsgemäß eine Absorption, in einem Lösungsmittelgemisch LM-II wobei eine Zusammensetzung Z-B und ein an den absorbierten Gasen verarmter Abgasstrom erhalten wird.

Die Zusammensetzung Z-B umfasst das Lösungsmittelgemisch LM-II und die darin absorbierten Gase.

Sofern als Lösungsmittelgemisch LM-II Wasser eingesetzt wird, enthält die Zusammensetzung Z-B beispielsweise 90,0 bis 99,9999 Gew.-% Wasser, insbesondere 95,0 bis 99,999 Gew.-%, bevorzugt 98,0 bis 99,99 Gew.-% Wasser; beispielsweise 0,01 bis 2,5 Gew.-% Distickstoffmonoxid, insbesondere 0,1 bis 1,5 Gew.-%, bevorzugt 0,5 bis 1,0 Gew.-% Distickstoffmonoxid; beispielsweise 0,001 bis 0,5 Gew.-% Kohlenstoffdioxid, insbesondere 0,01 bis 0,25 Gew.-% Kohlenstoffdioxid; beispielsweise 0,0001 bis 0,1 Gew.-% Stickstoff, insbesondere 0,001 bis 0,05 Gew.-% Stickstoff; sowie Spuren von Sauerstoff und Argon. Dabei ergibt die Summe der Komponenten der Zusammensetzung Z-B 100 Gew.-%.

Vorzugsweise wird Schritt (iii) des erfindungsgemäßen Verfahrens kontinuierlich durchgeführt. Das heißt im Rahmen der vorliegenden Erfindung, dass kontinuierlich das Lösungsmittelgemisch LM-II und das Gasgemisch G-1 in Kontakt gebracht werden, wobei sich kontinuierlich die Zusammensetzung Z-B und der verarmte Abgasstrom bilden.

Vorzugsweise werden die Schritte (i) und (iii) des erfindungsgemäßen Verfahrens kontinuierlich durchgeführt.

Erfindungsgemäß werden bei der Absorption gemäß Schritt (iii) vorzugsweise Distickstoffmonoxid und Kohlenstoffdioxid absorbiert. Auch im Gasgemisch G-1 verbleibende Stickoxide NOₓ werden gemäß Schritt (iii) absorbiert.

Vom eintretenden Gasstrom werden in Schritt (iii) erfindungsgemäß vorzugsweise 60 bis 80 % absorbiert.

Das erfindungsgemäße Verfahren umfasst vorzugsweise weiter einen Schritt (iv), bei dem ein Gasgemisch G-2 aus der Zusammensetzung Z-B unter Erhalt eines Lösungsmittelgemischs LM-II' desorbiert wird.

Dabei werden gemäß Schritt (iv) vorzugsweise Distickstoffmonoxid und Kohlenstoffdioxid aus der Zusammensetzung Z-B desorbiert.

Das Lösungsmittelgemisch LM-II' enthält neben dem eingesetzten Lösungsmittelgemisch LM-II noch nicht desorbierte Gase und Folgeprodukte.

Das erhaltene Gasgemisch G-2 enthält mindestens 50 Vol.-% N₂O, besonders bevorzugt mindestens 60 Vol.-% N₂O und ganz besonders bevorzugt mindestens 75 Vol.-% N₂O. Üblicherweise enthält das Gasgemisch G-2 bis zu 99 Vol.-% N₂O, insbesondere bis zu 97 Vol.-% N₂O, beispielsweise bis zu 96 Vol.-% N₂O, bis zu 95 Vol.-% N₂O, bis zu 94 Vol.-% N₂O, bis zu 93 Vol.-% N₂O, bis zu 92 Vol.-% N₂O, bis zu 91 Vol.-% N₂O, bis zu 90 Vol.-% N₂O oder auch bis zu 85 Vol.-% N₂O.

Im Rahmen der vorliegenden Erfindung hat das Gasgemisch G-2 beispielsweise einen Gehalt an N₂O von 60 bis 95 Vol.-%, vorzugsweise von 70 bis 90 Vol.-%, insbesondere von 75 bis 85 Vol.-%, besonders bevorzugt beispielsweise 76 Vol.-%, 77 Vol.-%, 78 Vol.-%, 79 Vol.-%, 80 Vol.-%, 81 Vol.-%, 82 Vol.-%, 83 Vol.-%, 84 Vol.-% oder 85 Vol.-%.

Das Gasgemisch G-2 hat beispielsweise einen Gehalt an CO₂ von 1 bis 20 Vol.-%, vorzugsweise von 5 bis 15 Vol.-%, besonders bevorzugt beispielsweise 6 Vol.-%, 7 Vol.-%, 8 Vol.-%, 9 Vol.-%, 10 Vol.-%, 11 Vol.-%, 12 Vol.-%, 13 Vol.-% oder 14 Vol.-%. Gleichzeitig hat das Gasgemisch G-2 beispielsweise einen Gehalt an O₂ von 0,01 bis 5,0 Vol.-%, vorzugsweise von 0,1 bis 2,5 Vol.-%, besonders bevorzugt beispielsweise 0,2 bis 1,0 Vol.-%. Darüber hinaus kann das Gasgemisch G-2 noch 0,1 bis 10 Vol.-% N₂ enthalten, vorzugsweise 0,5 bis 5 Vol.-%, sowie weitere Komponenten, beispielsweise Stickoxide oder Lösungsmittelreste. Gleichzeitig enthält das Gasgemisch G-2 weniger als 1 Vol.-% O₂, insbesondere weniger als 0,5 Vol.-% O₂, weniger als 0,5 Vol.-% NOₓ. NOₓ kann dabei beispielsweise in einer Menge von 0 bis 0,1 Vol.-% enthalten sein, vorzugsweise 0,0001 bis 0,01 Vol.-%, besonders bevorzugt 0,0002 bis 0,02 Vol.-%. Dabei ergibt die Summe der Komponenten des Gasgemischs G-2 100 Vol.-%.

Sofern der Schritt (A) nach dem Schritt (iv) keine weiteren Schritte umfasst, entspricht die Zusammensetzung des Gasgemischs G-A der Zusammensetzung des Gasgemischs G-2.

Gemäß einer bevorzugten Ausführungsform des erfindungsgemäßen Verfahrens können sowohl das Lösungsmittelgemisch LM-I' als auch das Lösungsmittelgemisch LM-II' zumindest teilweise in das erfindungsgemäße Verfahren zurückgeführt werden.

Dabei ist es erfindungsgemäß möglich, dass das Lösungsmittelgemisch LM-I' und/oder das Lösungsmittelgemisch LM-II' zumindest teilweise als Lösungsmittelgemisch LM-I oder LM-II in das erfindungsgemäße Verfahren zurückgeführt werden. Erfindungsgemäß kann das Lösungsmittelgemisch LM-I' und/oder das Lösungsmittelgemisch LM-II' insbesondere behandelt werden, bevor es als Lösungsmittelgemisch LM-I oder LM-II wieder in das Verfahren eingesetzt wird.

Insbesondere ist es erfindungsgemäß auch möglich, dass nur ein Teil des Lösungsmittelgemischs LM-I' und/oder LM-II' wieder in das Verfahren eingesetzt wird und beispielsweise mit Wasser oder einem anderen Lösungsmittel versetzt wird, um dann als Lösungsmittelgemisch LM-I und/oder LM-II wieder in das Verfahren eingesetzt zu werden.

Die Absorption gemäß Schritt (i) bzw. (iii) des erfindungsgemäßen Verfahrens kann grundsätzlich nach allen dem Fachmann bekannten Verfahren erfolgen. Insbesondere kann die Absorption im Lösungsmittelgemisch durch Erhöhung des Drucks des Eduktgases oder durch Absenkung der Temperatur des Lösungsmittelgemischs oder durch eine Kombination der genannten Maßnahmen herbeigeführt werden.

Vorzugsweise wird bei Schritt (i) bzw. (iii) des erfindungsgemäßen Verfahrens zunächst das Gasgemisch komprimiert, beispielsweise auf einen Druck von 10 bis 35 bar, bevorzugt von 13 bis 30 bar, vorzugsweise von 14 bis 25 bar. Anschließend wird das komprimierte Gasgemisch vorzugsweise bei diesem Druck mit dem Lösungsmittelgemisch LM-I gemäß Schritt (i) oder in dem Lösungsmittelgemisch LM-II gemäß Schritt (iii) in Kontakt gebracht.

Daher betrifft die vorliegende Erfindung auch ein wie zuvor beschriebenes Verfahren zur Reinigung eines Gasgemischs G-0 enthaltend Distickstoffmonoxid, wobei der Druck bei der Absorption gemäß Schritt (i) oder (iii) oder (i) und (iii) in einem Bereich von 10 bis 35 bar liegt.

Die Absorption gemäß Schritt (i) und Schritt (iii) erfolgt erfindungsgemäß in Einrichtungen (Absorber), in denen eine Gas-Flüssig-Phasengrenzfläche erzeugt wird, über die ein Stoff- und Wärmeübergang zwischen den Phasen ermöglicht wird, und die bei Bedarf mit internen oder externen Einrichtungen zur Wärmezufuhr und/oder Wärmeabfuhr versehen sind.

Die Führung der Phasen im Absorber kann im Gleichstrom, im Gegenstrom oder einer Kombination der genannten erfolgen.

Die Absorption kann erfindungsgemäß ein- oder mehrstufig durchgeführt werden, vorzugsweise einstufig. Dabei wird bei der Absorption vorzugsweise als Absorber eine Einrichtung mit mehreren theoretischen Trennstufen verwendet, insbesondere 2 bis 8 theoretischen Trennstufen, besonders bevorzugt 3 bis 6.

Mögliche Ausführungsformen des Absorbers sind jeweils Kolonnen mit Böden, beispielsweise Glockenböden oder Siebböden, Kolonnen mit strukturierten Einbauten, wie beispielsweise Packungen, Kolonnen mit unstrukturierten Einbauten, wie beispielsweise Füllkörpern, oder Apparate in denen die Flüssigphase dispergiert vorliegt, beispielsweise durch Versprühen in Düsen, oder eine Kombination der genannten.

Die Desorption des Gasgemischs G-1 bzw. G-2 aus der Zusammensetzung Z-A bzw. Zusammensetzung Z-B gemäß Schritt (ii) oder (iv) des erfindungsgemäßen Verfahrens kann durch Druckabsenkung über dem Lösungsmittelgemisch, Temperaturerhöhung des Lösungsmittelgemischs oder durch Strippung mit Lösungsmitteldampf oder einer Kombination der genannten herbeigeführt werden.

Die Anforderungen an die Einrichtungen (Desorber) zur Desorption des Gasgemischs G-1 bzw. G-2 aus der Zusammensetzung Z-A bzw. Zusammensetzung Z-B, sowie die Führung der Phasen sind analog zu denen des Absorbers, d.h. geeignet sind Einrichtungen, in denen eine Gas-Flüssig-Phasengrenzfläche erzeugt wird, über die ein Stoff - und Wärmeübergang zwischen den Phasen ermöglicht wird, und die bei Bedarf mit internen oder externen Einrichtungen zur Wärmezufuhr und/oder Wärmeabfuhr versehen sind.

Die Desorption kann erfindungsgemäß ein- oder mehrstufig durchgeführt werden.

Mögliche Ausführungsformen des Desorbers sind ein einfacher (Entspannungs-)Behälter und Kolonnen.

Eine bevorzugte Ausführungsform der vorliegenden Erfindung, in der die Absorption, also das Inkontaktbringen mit dem Lösungsmittelgemisch, und die Desorption apparativ vereint sind, ist beispielsweise die Trennwandkolonne. Dabei wird das Inkontaktbringen, und damit verbunden die Absorption, und die Desorption durch Temperaturwechsel mehrstufig im Gegenstrom betrieben kombiniert mit einer Strippung mit Lösungsmitteldampf. Dabei kann sowohl gemäß (i) und (ii) als auch gemäß (iii) und (iv) eine apparative Vereinigung der Absorption und der Desorption erfolgen, insbesondere in einer Trennwandkolonne.

In einer bevorzugten Ausführungsform betrifft die vorliegende Erfindung daher ein Verfahren wie oben beschrieben, wobei die Schritte (i) und (ii) oder die Schritte (iii) ünd (iv) oder die Schritte (i) und (ii) und die Schritte (iii) und (iv) in einer Trennwandkolonne durchgeführt werden.

Im Sinne einer besonders bevorzugten Ausführungsform der Erfindung wird gemäß Schritt (i) zunächst das Gasgemisch G-0 enthaltend N₂O unter erhöhtem Druck p_{Abso} in einer im Gegenstrom betriebenen Absorptionskolonne mit Füllkörperschüttung mit dem Lösungsmittelgemisch LM-I in Kontakt gebracht, wobei eine Absorption stattfinden kann, und eine Zusammensetzung Z-A erhalten wird. Gemäß Schritt (ii) wird die Zusammensetzung Z-A gemäß dieser Ausführungsform in einen Behälter überführt, in dem die Zusammensetzung Z-A auf einen niedrigeren Druck p_{Deso} < p_{Abso} entspannt wird. Der Prozess wird vorzugsweise nahezu isotherm betrieben mit einer Temperaturdifferenz zwischen Absorptions- und Desorptionstemperatur von maximal 20 K, vorzugsweise maximal 15 K, insbesondere maximal 10 K. Der Absorptionsdruck beträgt hierbei 1 bis 100 bar, bevorzugt 5 bis 65 bar, insbesondere 10 bis 40 bar, bevorzugt 10 bis 35 bar, besonders bevorzugt 13 bis 30 bar, weiter bevorzugt etwa 14 bis 25 bar und der Desorptionsdruck 0,1 bis 2 bar absolut, vorzugsweise 0,5 bis 1,5 bar absolut, besonders bevorzugt 1,0 bis 1,2 bar absolut.

Vorzugsweise wird ebenfalls gemäß Schritt (iii) zunächst das Gasgemisch G-1 unter erhöhtem Druck p_{Abso} in einer im Gegenstrom betriebenen Absorptionskolonne mit Füllkörperschüttung mit einem Lösungsmittelgemisch LM-II in Kontakt gebracht, wobei die Zusammensetzung Z-B erhalten wird. Zusammensetzung Z-B wird gemäß Schritt (iv) in einen Behälter überführt, in dem die Zusammensetzung Z-B auf einen niedrigeren Druck p_{Deso} < p_{Abso} entspannt wird. Der Prozess wird vorzugsweise ebenfalls nahezu isotherm betrieben mit einer Temperaturdifferenz zwischen Absorptions- und Desorptionstemperatur von maximal 20 K, vorzugsweise maximal 15 K, insbesondere maximal 10 K. Der Absorptionsdruck beträgt hierbei 1 bis 100 bar, bevorzugt 5 bis 65 bar, insbesondere 10 bis 40 bar, bevorzugt 10 bis 35 bar, besonders bevorzugt 13 bis 30 bar, weiter bevorzugt etwa 14 bis 25 bar und der Desorptionsdruck 0,1 bis 2 bar absolut, vorzugsweise 0,5 bis 1,5 bar absolut, besonders bevorzugt 1,0 bis 1,2 bar absolut.

Erfindungsgemäß ist es wie zuvor beschrieben möglich, dass das Lösungsmittelgemisch LM-I' zumindest teilweise als Lösungsmittelgemisch LM-I in das Verfahren zurückgeführt wird oder dass das Lösungsmittelgemisch LM-II' zumindest teilweise als Lösungsmittelgemisch LM-II in das Verfahren zurückgeführt wird. Dabei kann das Lösungsmittelgemisch LM-I' bzw. LM-II' vollständig oder teilweise, bevorzugt teilweise als Lösungsmittelgemisch LM-I oder LM-II wieder eingesetzt werden.

Erfindungsgemäß ist es insbesondere bevorzugt, dass ein Teil des Lösungsmittelgemischs LM-I' bzw. LM-II' aus dem Verfahren ausgeschleust wird. Erfindungsgemäß können beispielsweise 0,01 bis 5 % des gesamten Lösungsmittelgemischs ausgeschleust werden, bevorzugt 0,05 bis 2,5 %, insbesondere 0,1 bis 1,0 %, beispielsweise 0,2 %, 0,3 %, 0,4 %, 0,5 %, 0,6 %, 0,7 %, 0,8 % oder 0,9 %.

Erfindungsgemäß ist es bevorzugt, dass das Lösungsmittelgemisch LM-I' bzw. LM-II' teilweise zurückgeführt wird, wobei jeweils andere Verbindungen, insbesondere zusätzliches Lösungsmittel, beispielsweise Wasser, zu dem Lösungsmittelgemisch zugesetzt werden können. Dabei werden andere Verbindungen in einer Menge zugesetzt, die der Menge des Lösungsmittelgemischs LM-I' bzw. LM-II' entspricht, die nicht in das Verfahren zurückgeführt wird.

Erfindungsgemäß ist es auch möglich, das Lösungsmittelgemisch LM-I' bzw. LM-II' vollständig als Lösungsmittelgemisch LM-I oder LM-II wieder einzusetzen. Dabei ist es erfindungsgemäß insbesondere möglich, dass durch geeignete Maßnahmen einzelne Verbindungen aus dem Lösungsmittelgemisch LM-I' oder LM-II' entfernt werden, beispielsweise Salze oder Lösungsmittel, bevor das jeweilige Gemisch als Lösungsmittelgemisch LM-I oder LM-II in das Verfahren zurückgeführt wird.

Daher betrifft die vorliegende Erfindung auch ein wie zuvor beschriebenes Verfahren zur Reinigung eines Gasgemischs G-0 enthaltend Distickstoffmonoxid, wobei das Lösungsmittelgemisch LM-I' zumindest teilweise als Lösungsmittelgemisch LM-I in das Verfahren zurückgeführt wird, oder wobei das Lösungsmittelgemisch LM-II' zumindest teilweise als Lösungsmittelgemisch LM-II in das Verfahren zurückgeführt wird, oder wobei das Lösungsmittelgemisch LM-I' zumindest teilweise als Lösungsmittelgemisch LM-I in das Verfahren zurückgeführt wird und das Lösungsmittelgemisch LM-II' zumindest teilweise als Lösungsmittelgemisch LM-II in das Verfahren zurückgeführt wird.

Bei dieser Verfahrensführung wird das erfindungsgemäße Verfahren bevorzugt als kontinuierliches Verfahren ausgeübt.

Sofern als Lösungsmittelgemisch LM-I und als Lösungsmittegemisch LM-II Wasser eingesetzt wird, wird in einer besonders bevorzugten kontinuierlichen Ausführung des Verfahrens ein Teil des Lösungsmittelgemischs LM-I' bzw. LM-II' ausgeschleust und durch entsalztes Wasser ersetzt. Die Menge des Lösungsmittelgemischs, die auszuschleusen ist, und die Menge an frischem Lösungsmittel, insbesondere an Wasser, das zuzufügen ist, werden so bemessen, dass einerseits die Menge an Lösungsmittelgemisch LM-I bzw. LM-II im System annährend konstant bleibt und andererseits die Menge an gelösten Salzen im Lösungsmittelgemisch LM-I bzw. im Lösungsmittelgemisch LM-II vorzugsweise nicht über 10 Gew.-% ansteigt, besonders bevorzugt nicht über 7 Gew.-% und ganz besonders bevorzugt nicht über 5 Gew.-% jeweils bezogen auf das gesamte Lösungsmittelgemisch LM-I bzw. LM-II, ansteigt.

Neben den Schritten (i), (ii). (iii) und (iv) kann Schritt (A) des erfindungsgemäßen Verfahrens auch weitere Schritte umfassen. So kann das Verfahren beispielsweise auch eine weitere Behandlung des Gasgemischs G-1 zwischen den Schritten (ii) und (iii) umfassen. Derartige Behandlungen umfassen beispielsweise eine Änderung der Temperatur oder eine Änderung des Drucks oder eine Änderung der Temperatur und des Drucks.

Dabei kann sich beispielsweise die Zusammensetzung eines Gasgemischs ändern, beispielsweise durch Kondensation einer der Komponenten. Bei diesen Komponenten kann es sich beispielsweise um Wasser oder eine andere im Lösungsmittelgemisch LM-I enthaltene Verbindung handeln, vorzugsweise um ein Lösungsmittel, das im Rahmen des erfindungsgemäßen Verfahrens im Lösungsmittelgemisch LM-I für Schritt (i) eingesetzt wird.

Erfindungsgemäß ist es möglich, dass aus dem Gasgemisch G-1 oder G-2 weitere Komponenten abgetrennt werden. So ist es beispielsweise möglich, dass aus dem Gasgemisch G-2 durch Kompression und anschließendes Abkühlen Spuren von Wasser abgetrennt werden, die nach der Desorption gemäß Schritt (iv) im Gasgemisch G-2 enthalten sein können.

Dabei wird das Gasgemisch G-2 vorteilhafterweise auf einen Druck von 1 bis 35 bar komprimiert, bevorzugt 2 bis 30 bar, weiter bevorzugt 3 bis 27 bar. Eine Abkühlung erfolgt vorzugsweise anschließend, bevorzugt auf 1 bis 25 °C, besonders bevorzugt 3 bis 20 °C, insbesondere 4 bis 15°C, weiter bevorzugt 8 bis 12°C.

Im Rahmen der vorliegenden Erfindung wird nach Schritt (A) im Rahmen von Schritt (B) eine Kondensation des gemäß Schritt (A) erhaltenen Gasgemischs G-Ä durchgeführt.

Dabei wird eine flüssige Zusammensetzung Z-1 enthaltend Distickstoffmonoxid und ein gasförmiges Gemisch G-K erhalten, wobei das gasförmige Gemisch G-K in die Behandlung gemäß Schritt (A) zurückgeführt wird.

Die Kondensation gemäß Schritt (B) des erfindungsgemäßen Verfahrens kann grundsätzlich nach jedem dem Fachmann bekannten geeigneten Verfahren erfolgen. Dabei ist es im Rahmen der vorliegenden Erfindung bevorzugt, dass das Gasgemisch G-A zumindest teilweise kondensiert wird. Erfindungsgemäß werden dabei 20 bis 99 Gew.-%, bevorzugt 50 bis 90 Gew.-% und ganz besonders bevorzugt 60 bis 80 Gew.-% des Gasgemischs G-A kondensiert.

Daher betrifft die vorliegende Erfindung gemäß einer weiteren Ausführungsform ein Verfahren zur Reinigung eines Gasgemischs enthaltend Distickstoffmonoxid wie zuvor beschrieben, wobei in Schritt (B) 20 bis 90 Gew.-% des Gasgemischs G-A kondensiert werden.

Durch die Behandlung gemäß Schritt (B) des erfindungsgemäßen Verfahrens wird die flüssige Zusammensetzung Z-1 erhalten, bei der der Anteil störender Nebenkomponenten, insbesondere Sauerstoff, im Vergleich zum Gasgemisch G-A weiter verringert ist.

Gleichzeitig wird ein gasförmiges Gemisch G-K erhalten, das neben Spuren von Distickstoffmonoxid weitere Komponenten wie Sauerstoff oder Kohlenstoffmonoxid enthalten kann.

Erfindungsgemäß enthält das gasförmige Gemisch G-K beispielsweise 70 bis 90 Vol.-% Distickstoffmonoxid, insbesondere 75 bis 85 Vol.-%, besonders bevorzugt 78 bis 82 Vol.-%. Das gasförmige Gemisch G-K enthält erfindungsgemäß darüber hinaus beispielsweise 4 bis 18 Vol.-% Kohlenstoffdioxid, insbesondere 6 bis 16 Vol.-%, besonders bevorzugt 8 bis 12 Vol.-% CO₂ . Weiter enthält das gasförmige Gemisch G-K beispielsweise 0,01 bis 5 Vol.-% Sauerstoff, insbesondere 0,5 bis 3 Vol.-%, besonders bevorzugt 1,0 bis 2,0 Vol.-% Sauerstoff und beispielsweise 0 bis 1 Vol.-% Argon, wobei die Summe der Komponenten des gasförmigen Gemischs G-K 100 Vol.-% ergibt.

Vorzugsweise wird gemäß Schritt (B) das Gasgemisch G-A zunächst komprimiert und anschließend gekühlt. Dabei wird das Gasgemisch G-A vorteilhafterweise auf einen Druck von 1 bis 35 bar komprimiert, bevorzugt 2 bis 30 bar, weiter bevorzugt 3 bis 27 bar. Eine Abkühlung erfolgt vorzugsweise anschließend, bevorzugt auf 10 bis -70 °C, besonders bevorzugt 8 bis -30 °C, insbesondere 5 bis -25°C.

Sofern vor Schritt (B) des erfindungsgemäßen Verfahrens ein Schritt erfolgt, bei dem ein bereits komprimiertes Gasgemisch erhalten wird, umfasst Schritt (B) vorzugsweise keine weitere Kompression.

Das erfindungsgemäße Verfahren hat unter anderem den Vorteil, dass CO₂ neben der im Vergleich zu anderen Inertgasen guten Inertisierungswirkung eine dem N₂O sehr ähnliche Siedekurve und ähnliche kritische Parameter aufweist. Dadurch lässt sich das im erfindungsgemäßen Verfahren erhaltene Gasgemisch G-2 enthaltend N₂O und gegebenenfalls CO₂ bei einer höheren Temperatur kondensieren, als ein vergleichbares Gemisch aus N₂O und einem anderen Inertgas. Durch die sehr ähnlichen Siedekurven weist das kondensierte Gasgemisch fast das gleiche Verhältnis von N₂O zu CO₂ auf wie das gasförmige Gemisch, so dass das Inertisierungsmittel in der flüssigen Phase erhalten bleibt.

Darüber hinaus werden durch die Teilkondensation in Schritt (B) die leicht siedenden Komponenten und insbesondere O₂ als störende Komponente in Z-1 noch weiter abgereichert.

Erfindungsgemäß kann die durch das erfindungsgemäße Verfahren erhaltene flüssige Zusammensetzung Z-1 enthaltend Distickstoffmonoxid in einer weiteren Umsetzung eingesetzt werden.

Die flüssige Zusammensetzung Z-1 kann insbesondere vorteilhaft als Oxidationsmittel eingesetzt werden. Vorteilhafterweise enthält die flüssige Zusammensetzung Z-1 neben Distickstoffmonoxid auch Kohlenstoffdioxid. CO₂ wirkt inertisierend und gewährleistet einen sicherheitstechnisch unbedenklichen Betrieb bei der Aufbereitung und insbesondere bei der Lagerung und weiteren Verwendung der flüssigen Zusammensetzung Z-1. Es wurde gefunden, dass bei der Anwesenheit von CO₂ als Inertgas in Zusammensetzungen enthaltend N₂O im Vergleich zu anderen Inertgasen deutlich geringere Mengen Kohlenstoffdioxid benötigt werden, um die Selbstzerfallfähigkeit von Distickstoffmonoxid zu unterbinden. Damit reichen geringe Mengen an CO₂ zur Inertisierung der flüssigen Zusammensetzung Z-1 aus.

Erfindungsgemäß kann das Verfahren zur Reinigung eines Gasgemischs enthaltend Distickstoffmonoxid auch weitere Schritte umfassen. So ist es auch möglich, dass das Verfahren weitere Schritte nach dem Schritt (B) umfasst.

Beispielsweise kann im Rahmen des erfindungsgemäßen Verfahrens die Zusammensetzung Z-1 weiter behandelt werden. Dabei ist es im Rahmen der vorliegenden Erfindung insbesondere möglich, dass ein weiterer Schritt zur Aufkonzentrierung der Zusammensetzung Z-1 erfolgt. Dabei sind grundsätzlich alle dem Fachmann bekannten geeigneten Methoden zur weiteren Aufkonzentrierung der Zusammensetzung Z-1 oder zur Entfernung von Verunreinigungen wie beispielsweise von Resten von Lösungsmittel, möglich.

Erfindungsgemäß umfasst das Verfahren insbesondere einen weiteren Schritt (C) zur Entfernung von Verunreinigungen aus der Zusammensetzung Z-1. Dabei wird vorzugsweise gemäß Schritt (C) die Zusammensetzung Z-1 enthaltend Distickstoffmonoxid mit einem Gasgemisch S-1 unter Erhalt einer Zusammensetzung Z-2 und eines Gasgemischs S-2 in Kontakt gebracht.

Daher betrifft die vorliegende Erfindung gemäß einer weiteren Ausführungsform auch ein wie zuvor beschriebenes Verfahren zur Reinigung eines Gasgemischs enthaltend Distickstoffmonoxid, wobei das Verfahren die Schritt (C) umfasst:
(C) Inkontaktbringen der Zusammensetzung Z-1 enthaltend Distickstoffmonoxid mit einem Gasgemisch S-1 unter Erhalt einer Zusammensetzung Z-2 und eines Gasgemischs S-2.

Mittels der Behandlung gemäß Schritt (C) des erfindungsgemäßen Verfahrens ist es möglich, weitere Verunreinigungen aus der flüssigen Zusammensetzung Z-1 zu entfernen, die bei einer weiteren Umsetzung stören könnten, beispielsweise Sauerstoff.

Als Gasgemisch S-1 können prinzipiell alle Substanzen eingesetzt werden die einen niedrigeren Siedepunkt als Distickstoffmonoxid haben oder Gemische davon. Bevorzugt werden Gase eingesetzt, die nicht mit Distickstoffmonoxid reagieren, beispielsweise Stickstoff, Helium, Neon, Argon, Krypton, Xenon, Wasserstoff, Kohlenstoffmonoxid, Methan und Tetrafluormethan. Bevorzugt wird erfindungsgemäß Stickstoff eingesetzt.

Daher betrifft die vorliegende Erfindung gemäß einer weiteren Ausführungsform auch ein wie zuvor beschriebenes Verfahren zur Reinigung eines Gasgemischs enthaltend Distickstoffmonoxid, wobei das Gasgemisch S-1 ausgewählt ist aus der Gruppe bestehend aus Stickstoff, Helium, Neon, Argon, Krypton, Xenon, Wasserstoff, Kohlenstoffmonoxid, Methan und Tetrafluormethan.

Für die Behandlung gemäß Schritt (C) kann im Rahmen der vorliegenden Erfindung jeder Apparat verwendet werden, der dazu geeignet ist Gase und Flüssigkeiten miteinander in Kontakt zu bringen. Als Beispiele sind hier Blasensäulen, beispielsweise in Gleich- oder Gegenstrom betrieben, mit oder ohne Füllkörper bzw. Packung, in Riesel - oder Sumpf-Fahrweise, Rührkessel, beispielsweise mit Begasungsrührer, oder ähnliche zu nennen. Die Behandlung gemäß Schritt (C) kann sowohl in Batch oder kontinuierlich erfolgen. Bevorzugt wird sie allerdings kontinuierlich durchgeführt.

Die Behandlung gemäß Schritt (C) wird vorzugsweise bei einer Temperatur zwischen - 90°C und +37°C, bevorzugt bei einer Temperatur zwischen -80°C und 0°C durchgeführt. Bevorzugt wird die Behandlung gemäß Schritt (C) bei einem Druck durchgeführt, der mindestens so hoch ist wie der Dampfdruck der flüssigen Zusammensetzung Z-1 bei der gewählten Temperatur und maximal bei 100 bar. Bevorzugt wird ein Druck gewählt, der 0,2 bis 5 bar oberhalb des Druckes, bei dem die Absorption betrieben wird, liegt.

Die Menge an eingesetztem Gasgemisch S-1 muss groß genug sein, um die gewünschte Sauerstoff-Abreicherung zu erreichen, aber andererseits so klein wie möglich, um Verluste von Distickstoffmonoxid zu vermeiden. Typischerweise werden zwischen 5 und 100 mol Gasgemisch S-1 pro mol Sauerstoff in der flüssigen Zusammensetzung Z-1 eingesetzt, bevorzugt zwischen 15 und 30 mol Gasgemisch S-1 pro mol Sauerstoff in der flüssigen Zusammensetzung Z-1 eingesetzt.

Dabei wird gemäß Schritt (C) eine flüssige Zusammensetzung Z-2 erhalten, deren Gehalt an Sauerstoff gegenüber der flüssigen Zusammensetzung Z-1 weiter vermindert ist.

Erfindungsgemäß enthält die Zusammensetzung Z-2 beispielsweise 75 bis 95 Vol.-% Distickstoffmonoxid, insbesondere 80 bis 90 Vol.-%, besonders bevorzugt 82 bis 88 Vol.-%. Die Zusammensetzung Z-2 enthält erfindungsgemäß darüber hinaus beispielsweise 4 bis 18 Vol.-% Kohlenstoffdioxid, insbesondere 6 bis 16 Vol.-%, besonders bevorzugt 8 bis 12 Vol.-% CO₂ . Weiter enthält die Zusammensetzung Z-2 beispielsweise 0,01 bis 1,0 Vol.-% Sauerstoff, insbesondere 0,05 bis 0,5 Vol.-%, besonders bevorzugt 0,1 bis 0,4 Vol.-% Sauerstoff und beispielsweise 0 bis 1 Vol.-% Stickstoff, wobei die Summe der Komponenten der Zusammensetzung Z-2 100 Vol.-% ergibt.

In Schritt (C) wird weiter ein Gasgemisch S-2 erhalten, das neben dem Gasgemisch S-1 weitere Komponenten enthalten kann, beispielsweise Sauerstoff.

Erfindungsgemäß enthält das Gasgemisch S-2 beispielsweise 70 bis 90 Vol.-% Distickstoffmonoxid, insbesondere 75 bis 85 Vol.-%, besonders bevorzugt 77 bis 82 Vol.-%. Das Gasgemisch S-2 enthält erfindungsgemäß darüber hinaus beispielsweise 4 bis 18 Vol.-% Kohlenstoffdioxid, insbesondere 6 bis 16 Vol.-%, besonders bevorzugt 8 bis 12 Vol.-% CO₂. Das Gasgemisch enthält beispielsweise 4 bis 18 Vol.-% Stickstoff, insbesondere 6 bis 16 Vol.-%, besonders bevorzugt 8 bis 12 Vol.-% Stickstoff. Weiter enthält das Gasgemisch S-2 beispielsweise 0,01 bis 5 Vol.-% Sauerstoff, insbesondere 0,5 bis 3 Vol.-%, besonders bevorzugt 1,0 bis 2,0 Vol.-% Sauerstoff und beispielsweise 0 bis 1 Vol.-% Argon, wobei die Summe der Komponenten des Gasgemischs S-2 100 Vol.-% ergibt.

Erfindungsgemäß ist es möglich, dass das Gasgemisch S-2 in eine Stufe des Verfahrens zurückgeführt wird. Gemäß einer derartigen Ausführungsform kann Distickstoffmonoxid, das in dem Gasgemisch S-2 enthalten sind, in das Verfahren zurückgeführt werden, um Ausbeuteverluste zu vermeiden.

Daher betrifft die vorliegende Erfindung gemäß einer weiteren Ausführungsform auch ein wie zuvor beschriebenes Verfahren zur Reinigung eines Gasgemischs enthaltend Distickstoffmonoxid, wobei das Gasgemisch S-2 in den Schritt (A) zurückgeführt wird.

Wie zuvor beschrieben, wird das Gasgemisch S-2 vorzugsweise in den Schritt (A) des erfindungsgemäßen Verfahrens zurückgeführt. Dabei wird im Rahmen der vorliegenden Erfindung das Gasgemisch S-2 mit einem anderen Gasgemisch gemischt. Vorzugsweise wird dabei das Gasgemisch S-2 derart in den Schritt (A) zurückgeführt, dass eine Gewinnung des gegebenenfalls im Gasgemisch S-2 enthaltenen Distickstoffmonoxids möglich ist. Daher ist es im Rahmen der vorliegenden Erfindung bevorzugt, dass das Gasgemisch S-2 mit einem Gasgemisch gemischt wird, das einer Absorption zugeführt wird, insbesondere dem Gasgemisch G-0 oder dem Gasgemisch G-1. Somit ist es im Rahmen der vorliegenden Erfindung bevorzugt, das Gasgemisch S-2 in Schritt (i) oder in Schritt (iii) des Schritts (A) zurückzuführen.

Daher betrifft die vorliegende Erfindung gemäß einer weiteren Ausführungsform auch ein wie zuvor beschriebenes Verfahren zur Reinigung eines Gasgemischs enthaltend Distickstoffmonoxid, wobei das Gasgemisch S-2 in Schritt (i) oder in Schritt (iii) des Schritts (A) zurückgeführt wird.

Die nach dem erfindungsgemäßen Verfahren erhaltene flüssige Zusammensetzung Z-1 oder Z-2 enthaltend Distickstoffmonoxid kann grundsätzlich für alle Anwendungen eingesetzt werden, bei denen üblicherweise reine Distickstoffmonoxidströme oder mit Inertgas versetzte Distickstoffmonoxidströme eingesetzt werden. Insbesondere eignen sich die Zusammensetzung Z-1 oder Z-2 beispielsweise für die Oxidation von Methanol zu Formaldehyd wie beispielsweise in der EP-A 0 624 565 oder DE-A 196 05 211 beschrieben. Daher betrifft die vorliegende Erfindung auch die Verwendung der nach einem erfindungsgemäßen Verfahren erhältlichen flüssigen Zusammensetzung Z-1 enthaltend Distickstoffmonoxid als Oxidationsmittel für Methanol.

Durch das erfindungsgemäße Verfahren werden flüssige Zusammensetzungen enthaltend Distickstoffmonoxid erhalten, die einen besonders geringen Anteil an störenden Nebenkomponenten enthalten. Dies ist insbesondere für die Verwendung als Oxidationsmittel vorteilhaft, da durch den geringen Anteil an störenden Nebenkomponenten kaum Nebenreaktionen auftreten und somit bei einer Oxidation besonders reine Produkte erhalten werden können. Vorzugsweise enthält die flüssige Zusammensetzung Z-1 nach der erfindungsgemäßen Reinigung neben Distickstoffmonoxid auch Kohlenstoffdioxid in geeigneten Mengen.

Die erfindungsgemäß gereinigte flüssige Zusammensetzung Z-1 enthält vorzugsweise 50 bis 99,0 Vol.-% Distickstoffmonoxid, 1 bis 20 Vol.-% Kohlenstoffdioxid und 0 bis 25 Vol.-% weitere Gase. Die angegebenen Vol.-% beziehen sich jeweils auf die gesamte Zusammensetzung Z-1. Die Summe der einzelnen Komponenten der Zusammensetzung Z-1 ergibt dabei 100 Vol.-%.

Vorzugsweise enthält die erfindungsgemäß gereinigte Zusammensetzung Z-1 60 bis 95 Vol.-% Distickstoffmonoxid, insbesondere 70 bis 90 Vol.-%, besonders bevorzugt 75 bis 89 Vol.-% Distickstoffmonoxid.

Die erfindungsgemäß gereinigte Zusammensetzung Z-1 enthält weiterhin 1 bis 20 Vol.-% Kohlenstoffdioxid. Vorzugsweise enthält die Zusammensetzung Z-1 5 bis 15 Vol.-% Kohlenstoffdioxid, insbesondere 6 bis 14 Vol.-% Kohlenstoffdioxid.

Vorzugsweise enthält die Zusammensetzung Z-1 0 bis 25 Vol.-%, besonders bevorzugt 0 bits 5 Vol.-% weitere Gase. Die erfindungsgemäß gereinigte Zusammensetzung Z-1 kann ein oder mehrere weitere Gase enthalten, wobei die angegebene Menge auf die Summe der enthaltenen Gase bezogen ist. Die Zusammensetzung Z-1 kann beispielsweise Sauerstoff, Stickstoff, Argon und Wasser enthalten.

Es wurde gefunden, dass bei der Anwesenheit von CO₂ als Inertgas in verflüssigten Gasgemischen enthaltend N₂O im Vergleich zu anderen Inertgasen deutlich geringere Mengen des Inertgases, also Kohlenstoffdioxid, benötigt werden, um einen sicheren Betrieb zu gewährleisten, beispielsweise um einen Selbstzerfall von Distickstoffmonoxid zu unterbinden.

Des weikren wird die Verwendung einer flüssigen Zusammensetzung Z-1 erhältlich nach einem erfindungsgemäßen Verfahren wie oben beschrieben als Oxidationsmittel, insbesondere als Oxidationsmittel für Olefine, beschrieben.

Ferner wird auch die Verwendung einer Zusammensetzung enthaltend 50 bis 99,0 Vol.-% Distickstoffmonoxid, 1 bis 20 Vol.-% Kohlenstoffdioxid und 0 bis 25 Vol.-% weitere Gase als Oxidationsmittel, insbesondere als Oxidationsmittel für Olefine bescrieben.

Derartige Zusammensetzungen sind insbesondere durch das erfindungsgemäße Reinigungsverfahren erhältlich.

Grundsätzlich eignen sich die erfindungsgemäß erhältlichen flüssigen Zusammensetzungen enthaltend Distickstoffmonoxid zur Oxidation von Olefinen. Geeignete Olefine sind beispielsweise offenkettige oder cyclische Olefine mit einer oder mehreren Doppelbindungen. Weiter bevorzugt sind cyclische Olefine mit einer oder mehreren Doppelbindungen, beispielsweise Cyclopenten, Cyclohexen, Cyclohepten, Cycloocten, Cyclodecen, Cycloundecen, Cyclododecen, 1,4-Cyclohexadien, 1,5-Cyclooctadien, 1,6-Cyclodecadien, 1,6,11-Cyclopentadecatrien, 1,5,9,13-Cyclohexadecatetraen oder 1,5,9-Cyclododecatrien.

Des weikren bescrhreibt die vorliegende Erfindung auch eine wie zuvor beschriebene Verwendung als Oxidationsmittel für Olefine, wobei das Olefin ausgewählt ist aus der Gruppe bestehend aus Cyclopenten, Cyclododecen und 1,5,9-Cyclododecatrien.

Die angereicherte und gereinigte N₂O-haltige flüssige Zusammensetzung Z-1 eignet sich ganz besonders zur Oxidation von Olefinen zu Ketonen. Für diesen Zweck kann vorzugsweise die flüssige Zusammensetzung Z-1 direkt mit dem Olefin zur Reaktion gebracht werden.

Für derartige Anwendungen ist es vorteilhaft, wenn der Anteil an Inertgasen in der flüssigen Zusammensetzung Z-1 möglichst gering ist, da ansonsten das Reaktorvolumen unnötig vergrößert wird.

Gemäß der ober bescrhriebenen Verwendung als Oxidationsmittel, insbesondere für Olefine, kann die Oxidation generell gemäß sämtliche Verfahrensführungen erfolgen, bei denen die Oxidation, insbesondere des Olefins, stattfindet. Insbesondere sind sowohl kontinuierliche Verfahrensführungen und Fahrweisen der Umsetzung als auch Batch-Reaktion möglich. Die Reaktionsbedingungen für die Oxidation werden erfindungsgemäß so gewählt, dass eine Reaktion stattfindet. Druck und Temperatur können entsprechend gewählt werden.

Vorzugsweise liegt der Druck in einem Bereich bis 500 bar, beispielsweise 1 bis 320 bar, bevorzugt 10 bis 300 bar, insbesondere 90 bis 280 bar. Die Temperatur liegt vorzugsweise in einem Bereich von 180 bis 320°C, beispielsweise 200 bis 300°C, insbesondere 240 bis 290°C.

Dabei kann die Oxidation in Gegenwart eines geeigneten Lösungsmittels durchgeführt werden. Es ist erfindungsgemäß aber ebenso möglich, die Oxidation ohne den Zusatz eines Lösungsmittels durchzuführen.

Vorzugsweise wird die Oxidation erfindungsgemäß durch geeignete Wahl des Drucks und der Temperatur so geführt, dass in der Reaktionszone keine Gasphase auftritt.

Im Folgenden wird die Erfindung anhand von Beispielen näher erläutert.

### BEISPIELE

### Beispiel 1: Verfahren zur Isolierung und Reinigung von N₂O

Als Quelle für das N₂O wird das Abgas einer Salpetersäureanlage verwendet, das wiederum teilweise mit dem Abgas einer Adipinsäure-Anlage und teilweise mit reinem NO betrieben wird. Von diesem Abgas werden 26,2 t/h zunächst auf 25 bar komprimiert und auf 35°C abgekühlt. Das dabei kondensierende Wasser, das noch geringen Mengen Salpetersäure enthält, wird abgetrennt und entsorgt.

Der verbleibende komprimierte Gasstrom (26,1 t/h) enthält 86,4 Vol.-% N₂, 8,1 Vol.-% N₂O, 3,1 Vol.-% O₂ und 1,1 Vol.-% CO₂ als Hauptkomponenten. Dieser Strom wird am Boden einer Absorptionskolonne mit 22,7 m Höhe und 5,5 m Durchmesser, die mit Pall-Ringe gefüllt ist, eingespeist. Im Gegenstrom dazu werden von oben 2290 t/h Wasser mit einer Temperatur von 35°C eingespeist. Das nicht adsorbierte Gas wird über eine Entspannungsturbine wieder in dem Abgasstrang der Salpetersäureanlage entspannt.

Das beladene Absorptionsmittel wird über eine Entspannungsturbine in dem ersten Desorberturm auf 1,1 bar entspannt. Der Desorberturm hat einen Durchmesser von 3,6 m und eine Höhe von 11,1 m und ist mit Pallringe gefüllt. Das Wasser wird zurück in den Absorberturm gefördert. In diesem Kreislauf wird der pH-Wert durch Zugabe von 25 %-ige Natronlauge zwischen 6 und 7 (online gemessen mit kalibrierten Glaselektroden) gehalten. Im Schnitt werden ca. 44 kg/h Natronlauge verbraucht.

Um die Aufpegelung von Salzen (Natriumnitrit, Natriumnitat und Natriumhydrogencarbonat) zu verhindern, werden aus dem Wasserkreislauf 2 t/h gepurgt und durch frisches vollentsalztes Wasser ersetzt. Mit einem Wärmetauscher im Wasserkreislauf wird die Wassertemperatur geregelt.

Das am Kopf des ersten Desorberturms erhaltene Gas (2,45 t/h) enthält 59,5 Vol.-% N₂O 24,2 Vol.-% N₂, 7,5 Vol.-% CO₂, 5,2 Vol.-% H₂O und 3,0 Vol.-% O₂ als Hauptkomponenten. Dieses Gas wird wiederum auf 25 bar komprimiert und auf 35°C abgekühlt. Das dabei kondensierende Wasser wird abgetrennt und entsorgt. Der komprimierte Gasstrom wird dann zusammen mit den rückgeführten Gasströmen aus der Partialkondensation und der Strippung am Boden in einen zweiten Absorber gegeben. Dieser Absorber hat einen Durchmesser von 1,9 m und eine Höhe von 14,3 m und ist mit Pallringen gefüllt. Im Gegenstrom dazu wird Wasser (274 t/a mit einer Temperatur von 35°C) als Absorptionsmittel zu dem Absorber gegeben.

Das nicht absorbierte Gas wird entspannt und zusammen mit dem Abgas des ersten Absorbers in dem Abgasstrang der Salpetersäureanlage entspannt.

Das beladene Absorptionsmittel wird dann in dem zweiten Desorberturm auf 1,1 bar entspannt. Das Wasser wird zurück in den Absorberturm gefördert. Um ein Absinken des pH-Wertes zu verhindern, werden aus dem Wasserkreislauf 225 kg/h gepurgt und durch frisches vollentsalztes Wasser ersetzt. Mit einem Wärmetauscher im Wasserkreislauf wird die Wassertemperatur des Wassers geregelt.

Das am Kopf des zweiten Desorberturms erhaltene Gas (2,9 t/h) enthält 81,7 Vol.-% N₂O, 10,7 Vol.-% CO₂, 5,3 Vol.-% H₂O, 1,7 Vol.-% N₂, und 0,45 Vol.-% O₂ als Hauptkomponenten. Dieses Gas wird wiederum auf 26 bar komprimiert und auf 13°C abgekühlt. Das dabei kondensierende Wasser wird abgetrennt und entsorgt.

Der komprimierte Gasstrom (2,8 t/h) wird dann über einen aufrecht stehendes Rohrbündelwärmetauscher geleitet, der auf der Mantelseite mit einer gekühlten Wasser/Glykol-Mischung betrieben wird, wo er auf -12°C abgekühlt wird. Dabei kondensiert einen Strom (2060 kg/h), der 87,9 Vol.-% N₂O, 11,4 Vol.-% CO₂, 0,3 Vol.-% H₂O, 0,3 Vol.-% N₂, und 0,14 Vol.-% O₂ als Hauptkomponenten enthält.

Um die Rohre des Wärmetauschers aufzutauen, werden zwei parallele Wärmetauscher eingesetzt, die in A/B-Fahrweise betrieben werden. Um den Auftauvorgang zu beschleunigen, sind die Wärmetauscher mit einer elektrischen Heizung versehen. Der nicht kondensierte Anteil (790 kg/h) enthält 81,5 Vol.-% N₂O, 11,2 Vol.-% CO₂, 5,6 Vol.-% N₂ und 1,3 Vol.-% O₂ als Hauptkomponenten und wird wie bereits oben erwähnt zum Eingang des zweiten Absorbers zurückgeführt.

Der kondensierte Strom wird dann in einer Strippkolonne, die in Rieselfahrweise bei 26 bar betrieben wird, mit Stickstoff (4 kg/h, entspricht 19 mol N₂/mol O₂ im flüssigen N₂O Feed) im Gegenstrom gestrippt. Die Strippkolonne hat einen Durchmesser von 0,35 m und eine Höhe von 4,15 m und ist mit einer strukturierten Packung aus Metall (Packungslänge: 3 m) versehen mit einer spezifischen Oberfläche von 350 m²/m³. Das Strippgas am Kopf der Kolonne (260 kg/h) enthält 78,4 Vol.-% N₂O, 10,8 Vol.-% CO₂, 9,6 Vol.-% N₂, und 1,0 Vol.-% O₂ als Hauptkomponenten und wird wie bereits oben erwähnt zum Eingang des zweiten Absorbers zurückgeführt.

Das flüssige Produkt am Sumpf der Strippkolonne (1835 kg/h) enthält 86,7 Vol.-% N₂O, 11,1 Vol.-% CO₂ und 1,9 Vol.-% N₂ als Hauptkomponenten und nur noch 100 Vol.-ppm O₂.

Durch die Verwendung der Strippkolonne kann der Gehalt an O₂ im flüssigen N₂O um den Faktor 14 verringert werden. Das molare Verhältnis N₂O zu O₂ steigt dabei von 630 auf fast 7300 mol/mol. Durch die Rückführung des Strippgases in die zweite Absorptionskolonne bleibt trotzdem die lsolierungsausbeute hoch. Die Isolierungsausbeute für N₂O (bezogen auf das komprimierte Gas nach der Desorption) beträgt 96,2 %.

Das aufkonzentrierte und gereinigte N₂O kann beispielsweise für die Oxidation von Olefinen, z.B. von 1,5,9-Cyclododecadien, verwendet werden.

### Vergleichsbeispiel 2: Kondensation ohne Gasrückführung

Wenn bei der Kondensation des aufkonzentrierten N₂O Stroms das verbleibende Gas nicht in die zweite Absorption zurückgeführt würde, würde die Isolierungsausbeute für N₂O (bezogen auf das komprimierte Gas nach der Desorption) statt 96,2% nur noch 70,2% betragen.

Um die gleiche Menge an N₂O bereit zu stellen wären also deutlich größere Apparate notwendig.

### Vergleichsbeispiel 3: Kondensation ohne Gasrückführung mit gleicher Isolierungsausbeute (Druck)

Wenn die lsolierungsausbeute an N₂O (wie oben definiert) ohne Gasrückführung ähnlich hoch ausfallen soll, muss bei der Kondensation ein größerer Anteil des Eingangsgases kondensiert werden.

Dies kann durch Anhebung des Druckes bei der Kondensation und Beibehaltung der Kondensationstemperatur von -12°C, wie im erfindungsgemäßen Beispiel 1, erreicht werden. Hierfür muss der Druck auf 31 bar angehoben werden (statt 26 bar im erfindungsgemäßen Beispiel 1). Dabei kondensieren ca. 96% des im Feed enthaltenen N₂O. Man erhält einen flüssigen Strom, der N₂O (86,9 Vol.-%), CO₂ (11,4 Vol.-%), O₂ (0,35 Vol.-%) und N₂ (1,1 Vol.-%) als Hauptkomponenten enthält.

Diese Vorgehensweise hat gegenüber der erfindungsgemäßen Vorgehensweise mehrere Nachteile. Um den höheren Druck zu erreichen, sind ein teurerer Kompressor und teurere Kondensatoren notwendig. Weiter enthält der verflüssigte N₂O-haltige Gasstrom deutlich mehr Sauerstoff (fast 2,5-mal mehr als im erfindungsgemäßen Beispiel 1), als wenn nur eine Partialkondensation durchgeführt wird. Um diesen Sauerstoff zu entfernen, muss dann deutlich mehr Stickstoff in der nachfolgenden Strippung eingesetzt werden, was wiederum mit höheren Verlusten an N₂O über das Abgas verbunden ist.

### Vergleichsbeispiel 4: Kondensation ohne Gasrückführung mit gleicher Isolierungsausbeute (Temperatur)

Wenn die Isolierungsausbeute an N₂O (wie oben definiert) ohne Gasrückführung ähnlich hoch ausfallen soll, muss bei der Kondensation einen größeren Anteil des Eingangsgases kondensiert werden.

Dies kann auch durch Ansenkung der Kondensationstemperatur und Beibehaltung des Druckes von 26 bar, wie im erfindungsgemäßen Beispiel 1, erreicht werden. Hierfür muss die Kondensationstemperatur auf -19°C abgesenkt werden (statt -12°C im erfindungsgemäßen Beispiel 1). Dabei kondensieren ca. 96% des im Feed enthaltenen N₂O. Man erhält einen flüssigen Strom, der N₂O (87,0 Vol.-%), CO₂ (11,4 Vol.-%), O₂ (0,32 Vol.-%) und N₂ (1,0 Vol.-%) als Hauptkomponenten enthält.

Diese Vorgehensweise hat aber gegenüber der erfindungsgemäßen Vorgehensweise mehrere Nachteile. Um die niedrigere Temperatur zu erreichen, ist eine deutlich teurere Kältemaschine notwendig und die Kombination von hohem Druck und niedriger Temperatur stellt hohe Anforderungen an das Konstruktionsmaterial der Kondensatoren. Weiter enthält der verflüssigte N₂O-haltige Gasstrom deutlich mehr Sauerstoff (fast 2,3-mal mehr als im erfindungsgemäßen Beispiel 1), als wenn nur eine Partialkondensation durchgeführt wird. Um diesen Sauerstoff zu entfernen muss dann deutlich mehr Stickstoff in eine nachfolgende Strippung eingesetzt werden, was wiederum mit höheren Verlusten an N₂O über das Abgas verbunden ist.

### Beispiel 5: Einfluss von O₂ auf die Zersetzung von 1,5,9-Cyclododecatrien

Um den Einfluss von O₂ auf die Zersetzung von 1,5,9-Cyclododecatrien zu untersuchen, wurden 500 g technisches 1,5,9-Cyclododecatrien in einem 1000 ml Glaskolben der mit einem Magnetrührer, einem Gaseinleitungsrohr und einem Rückflusskühler versehen war, vorgelegt. Der Kolben wurde dann in einem Ölbad auf 180°C erhitzt, und mit einem Brooks-Massendurchflussmesser wurden 2 NI/h synthetische Luft durch das Gaseinleitungsrohr eingeleitet.

Die Abgasmenge und deren Zusammensetzung wurden am Ausgang bestimmt. Von der Flüssigkeit wurden außerdem in regelmäßigen Abständen Proben genommen und gaschromatographisch analysiert. Aus der Abgasanalyse wird einen O₂-Verbrauch von 11 mmol/h errechnet. Der Gehalt an 1,5,9-Cyclododecatrien in der Lösung nimmt mit 2 %-Punkte/h ab. Dies bedeutet, dass 1,1 mol 1,5,9-Cyclododecatrien pro mol O₂ zerstört werden. Dabei bilden sich, abgesehen von kleinen Mengen an dem Monoepoxid von 1,5,9-Cyclododecatrien, keine definierten Produkte, sondern lediglich polymere Beläge. Ein Kontrollversuch zeigte, dass wenn anstatt synthetischer Luft nur Stickstoff eingeperlt wird, keine Abnahme des Gehaltes an 1,5,9-Cyclododecatrien beobachtet wird.

Dieser Versuch zeigt, dass auch schon bei Temperaturen, die deutlich unterhalb der Temperatur liegen, die nötig ist um 1,5,9-Cyclododecatrien mit N₂O zu oxidieren (ca. 250°C), Sauerstoff mit 1,5,9-Cyclododecatrien reagiert. Dabei bilden sich polymere Ablagerungen die zu einer Verstopfung des Reaktors führen können. Es ist also sehr wichtig ein N₂O-haltiges Gasgemisch als Oxidationsmittel einzusetzen, das möglichst wenig O₂ enthält, um sowohl eine hohe Selektivität zu erhalten als auch um Ablagerungen im Reaktor zu vermeiden.

### Beispiel 6: Einfluss von O₂ auf die Zersetzung von 4,8-Cyclododecadienon

Um den Einfluss von O₂ auf die Zersetzung von 4,8-Cyclododecadienon (das Produkt aus der Oxidation von 1,5,9-Cyclododecatrien mit N₂O) zu untersuchen, wurden 500 g 4,8-Cyclododecadienon (ca. 98 %ig, als Isomerengemisch) in einem 1000 ml Glaskolben der mit einem Magnetrührer, einem Gaseinleitungsrohr und einem Rückflusskühler versehen war, vorgelegt. Der Kolben wurde dann in einem Ölbad auf 180°C erhitzt und mit einem Massendurchflussmesser wurden 2 NI/h synthetische Luft durch das Gaseinleitungsrohr eingeleitet.

Die Abgasmenge und deren Zusammensetzung wurden am Ausgang bestimmt. Von der Flüssigkeit wurden außerdem in regelmäßigen Abständen Proben genommen und gaschromatographisch analysiert. Aus der Abgasanalyse wird einen O₂-Verbrauch von 8 mmol/h errechnet. Der Gehalt an 4,8-Cyclododecadienon in der Lösung nimmt mit 1,6 %-Punkte/h ab. Dies bedeutet, dass 1,2 mol 4,8-Cyclododecadienon pro mol O₂ zerstört werden. Dabei bilden sich keine definierten Produkte aber auch keine polymere Beläge. Ein Kontrollversuch zeigte, dass wenn anstatt synthetischer Luft nur Stickstoff eingeperlt wird, keine Abnahme des Gehaltes an 4,8-Cyclododecadienon beobachtet wird.

Dieser Versuch zeigt, dass auch schon bei Temperaturen, die deutlich unterhalb der Temperatur liegen, die nötig ist um 1,5,9-Cyclododecatrien mit N₂O zu 4,8-Cyclododecadienon zu oxidieren (ca. 250°C), Sauerstoff mit 4,8-Cyclododecadienon reagiert (obwohl wie erwartet etwas langsamer als mit 1,5,9-Cyclododecatrien). Es ist also wichtig, ein N₂O-haltiges Gasgemisch als Oxidationsmittel einzusetzen, das möglichst wenig O₂ enthält, um eine hohe Selektivität zu erhalten.

### Beispiel 7: Oxidation von 1,5,9-Cyclododecatrien mit einem N₂O-haltigen Gasgemisch, das nur 200 ppm O₂ enthält

Für die kontinuierliche Oxidation von 1,5,9-Cyclododecatrien mit N₂O wurde ein Doppelmantelrohrreaktor verwendet, der aus 7 hintereinander geschalteten Doppelmantelrohrwendel besteht. Das Reaktionsrohr hat einen Innendurchmesser von 6 mm und jede Rohrwendel eine Länge von 5,32 m. Das gesamte Reaktionsvolumen beträgt demnach 1,05 Liter. Im Doppelmantel wird einen Wärmeträgeröl zirkuliert, dessen Temperatur mittels eines Thermostats auf 253°C konstant gehalten wird. Die Umlaufmenge des Wärmeträgeröls wird so gewählt, dass der Temperaturunterschied zwischen Öleingang und Ölausgang kleiner 2 K ist. Das Wärmeträgeröl wird dabei im Gleichstrom zu den Reaktanden geführt. Der Reaktor ist am Ausgang mit einem Druckregelventil versehen, das den Reaktionsdruck auf 100 bar konstant hält.

Die Edukte (1,5,9-Cyclododecatrien, kommerzielle Ware der Firma Degussa, und N₂O, medical grade der Firma Linde, enthält nach Analyse 200 ppm O₂) werden mittels geeigneter Dosierpumpen (Membrankolbenpumpen) gefördert und vor dem Reaktor noch bei Raumtemperatur in einem statischen Mischer gemischt, bevor sie den Reaktor erreichen. Die Feedmengen wurden so eingestellt, dass das molare Verhältnis zwischen 1,5,9-Cyclododecatrien und N₂O am Reaktoreingang 6,2 mol/mol beträgt, und die Verweilzeit (definiert als der Volumenstrom der Edukte bei Raumtemperatur und 100 bar geteilt durch den Reaktorvolumen) 0,65 Stunden beträgt. Die Reaktion wurde so lange durchgeführt, bis der Reaktor stationär war (ca. 6 Stunden), bevor mit der Bilanzierung begonnen wurde. Um die Fehler zu minimieren, betrug der Bilanzzeitraum immer 24 Stunden.

Nach dem Druckregelventil wird der Reaktoraustrag in einem gekühlten (etwa 20°C) Phasenscheider entspannt, und die Produkte (sowohl Gas als auch Flüssigkeit) analysiert. Der Umsatz an 1,5,9-Cyclododecatrien betrug 13,4%. Die Selektivität zu 4,8-Cyclododecadienon bezogen auf 1,5,9-Cyclododecatrien betrug 93,4%.

### Beispiel 8: Oxidation von 1,5,9-Cyclododecatrien mit einem N₂O-haltigen Gasgemisch, das 400 ppm O₂ und 8,3 Vol.-% CO₂ enthält

Beispiel 7 wurde wiederholt, wobei als Edukt ein Gasgemisch von der Firma Linde eingesetzt wurde, das 8,3 Vol.-% CO₂ und 400 ppm O₂ in N₂O enthielt.

Der Umsatz an 1,5,9-Cyclododecatrien betrug 13,4%. Die Selektivität zu 4,8-Cyclododecadienon bezogen auf 1,5,9-Cyclododecatrien betrug 93,8%.

Innerhalb der Messgenauigkeit haben also die Anwesenheit von CO₂ und die leicht erhöhte Menge noch keinen ausgeprägten Effekt auf die Reaktion.

### Beispiel 9: Oxidation von 1,5,9-Cyclododecatrien mit einem N₂O-haltigen Gasgemisch, das 1300 Vol.-ppm O₂ enthält

Beispiel 7 wurde wiederholt, wobei als Edukt ein Gasgemisch von der Firma Linde eingesetzt wurde, das 1300 Vol.-ppm O₂ in N₂O enthielt. Das entspricht in etwa dem Gehalt an Sauerstoff im flüssigen N₂O gemäß Beispiel 1, wenn auf einer Strippung verzichtet wird.

Die Reaktion konnte mit diesem Feed über 426 h stabil betrieben werden. Die Druckdifferenz über den Reaktor blieb dabei konstant. Zur Kontrolle wurde die erste Schlange abgebaut und in Abschnitte zersägt. An den Wänden hatte sich kein Polymer abgeschieden.

Der 1,5,9-Cyclododecatrien-Umsatz war mit 14,6% höher als gemäß Beispiel 8 (13,4%), wobei die Selektivität zu 4,8-Cyclododecadienon 91,5% betrug. Dies ist zwar fast 2 %-Punkte niedriger als die gemäß Beispiel 8 (93,8%) erreichte, aber immer noch in einem akzeptablen Bereich, zumal sich die Reaktion über längere Zeit stabil (d.h. ohne Bildung von Belägen) betreiben lässt.

### Vergleichsbeispiel 10: Oxidation von 1,5,9-Cyclododecatrien mit einem N₂O-haltigen Gasgemisch, das 2 Vol.-% O₂ enthält

Beispiel 7 wurde wiederholt, wobei als Edukt ein Gasgemisch von der Firma Linde eingesetzt wurde, das 2 Vol.-% O₂ in N₂O enthielt.

Die Reaktion war mit diesem Feed nicht stabil zu betreiben. Die Druckdifferenz über den Reaktor stieg kontinuierlich an, und nach 72 Stunden musste der Versuch abgebrochen werden, weil der Reaktor verstopft war. Die erste Schlange wurde daraufhin abgebaut und in Abschnitte zersägt. Es zeigte sich, dass zwischen 30 und 80 cm nach dem Reaktoreintritt das Rohr fast komplett mit polymeren Belägen verstopft war.

Mit derartig hohen Konzentrationen an O₂ in N₂O ist kein stabiler Betrieb der 1,5,9-Cyclododecatrien-Oxidation möglich.

## Patentansprüche

1. Verfahren zur Reinigung eines Gasgemischs G-0 enthaltend Distickstoffmonoxid mindestens umfassend die Schritte
(A) Behandeln des Gasgemischs G-0 enthaltend Distickstoffmonoxid unter Erhalt eines Gasgemischs G-A mindestens umfassend die Schritte
(i) Absorption des Gasgemischs G-0 in einem Lösungsmittelgemisch LM-I unter Erhalt eines Abgasstroms und einer Zusammensetzung Z-A
(ii) Desorption eines Gasgemischs G-1 aus der Zusammensetzung Z-A unter Erhalt eines Lösungsmittelgemischs LM-I'
(B) Kondensation des Gasgemischs G-A unter Erhalt einer flüssigen Zusammensetzung Z-1 enthaltend Distickstoffmonoxid und einem gasförmigen Gemisch G-K,
wobei das gasförmige Gemisch G-K in die Behandlung gemäß Schritt (A) zurückgeführt wird.

2. Verfahren zur Reinigung eines Gasgemischs enthaltend Distickstoffmonoxid nach Anspruch 1, wobei der Schritt (A) zusätzlich die Schritte (iii) und (iv) umfasst:
(iii) Absorption des Gasgemischs G-1 in einem Lösungsmittelgemisch LM-II. unter Erhalt eines Abgasstroms und einer Zusammensetzung Z-B
(iv) Desorption eines Gasgemischs G-2 aus der Zusammensetzung Z-B unter Erhalt eines Lösungsmittelgemischs LM-II'.

3. Verfahren zur Reinigung eines Gasgemischs enthaltend Distickstoffmonoxid nach einem der Ansprüche 1 oder 2, wobei der Schritt (A) weitere Schritte umfasst.

4. Verfahren zur Reinigung eines Gasgemischs enthaltend Distickstoffmonoxid nach einem der Ansprüche 1 bis 3, wobei das gasförmige Gemisch G-K in Schritt (i) oder in Schritt (iii) des Schritts (A) zurückgeführt wird.

5. Verfahren zur Reinigung eines Gasgemischs enthaltend Distickstoffmonoxid nach einem der Ansprüche 1 bis 4, wobei das Lösungsmittelgemisch LM-I oder das Lösungsmittelgemisch LM-II oder das Lösungsmittelgemisch LM-I und das Lösungsmittelgemisch LM-II ausgewählt ist aus der Gruppe bestehend aus organischen Lösungsmitteln und wässrigen Lösungsmittelgemischen.

6. Verfahren zur Reinigung eines Gasgemischs enthaltend Distickstoffmonoxid nach einem der Ansprüche 1 bis 5, wobei in Schritt (B) 20 bis 90 Gew.-% des Gasgemischs G-A kondensiert werden.

7. Verfahren zur Reinigung eines Gasgemischs enthaltend Distickstoffmonoxid nach einem der Ansprüche 1 bis 6, wobei das Verfahren Schritt (C) umfasst:
(C) Inkontaktbringen der Zusammensetzung Z-1 enthaltend Distickstoffmonoxid mit einem Gasgemisch S-1 unter Erhalt einer Zusammensetzung Z-2 und eines Gasgemischs S-2.

8. Verfahren zur Reinigung eines Gasgemischs enthaltend Distickstoffmonoxid nach Anspruch 7, wobei das Gasgemisch S-1 ausgewählt ist aus der Gruppe bestehend aus Stickstoff, Helium, Neon, Argon, Krypton, Xenon, Wasserstoff, Kohlenstoffmonoxid, Methan und Tetrafluormethan.

9. Verfahren zur Reinigung eines Gasgemischs enthaltend Distickstoffmonoxid nach einem der Ansprüche 7 oder 8, wobei das Gasgemisch S-2 in den Schritt (A) zurückgeführt wird.

10. Verfahren zur Reinigung eines Gasgemischs enthaltend Distickstoffmonoxid nach einem der Ansprüche 7 bis 9, wobei das Gasgemisch S-2 in Schritt (i) oder in Schritt (iii) des Schritts (A) zurückgeführt wird.

## Claims

1. A process for purifying a gas mixture G-0 comprising dinitrogen monoxide, at least comprising the steps of
(A) treating the gas mixture G-0 comprising dinitrogen monoxide to obtain a gas mixture G-A, at least comprising the steps of
(i) absorbing the gas mixture G-0 in a solvent mixture S-I to obtain an offgas stream and a composition C-A
(ii) desorbing a gas mixture G-1 from the composition C-A to obtain a solvent mixture S-I'
(B) condensing the gas mixture G-A to obtain a liquid composition C-1 comprising dinitrogen monoxide and a gaseous mixture G-K,
wherein the gaseous mixture G-K is recycled into the treatment in step (A).

2. The process for purifying a gas mixture comprising dinitrogen monoxide according to claim 1, wherein step (A) additionally comprises steps (iii) and (iv):
(iii) absorbing the gas mixture G-1 in a solvent mixture S-II to obtain an offgas stream and a composition C-B
(iv) desorbing a gas mixture G-2 from the composition C-B to obtain a solvent mixture S-II'.

3. The process for purifying a gas mixture comprising dinitrogen monoxide according to either of claims 1 and 2, wherein step (A) comprises further steps.

4. The process for purifying a gas mixture comprising dinitrogen monoxide according to any one of claims 1 to 3, wherein the gaseous mixture G-K is recycled into step (i) or into step (iii) of step (A).

5. The process for purifying a gas mixture comprising dinitrogen monoxide according to any one of claims 1 to 4, wherein the solvent mixture S-I or the solvent mixture S-II or the solvent mixture S-I and the solvent mixture S-II is/are selected from the group consisting of organic solvents and aqueous solvent mixtures.

6. The process for purifying a gas mixture comprising dinitrogen monoxide according to any one of claims 1 to 5, wherein from 20 to 90% by weight of the gas mixture G-A is condensed in step (B).

7. The process for purifying a gas mixture comprising dinitrogen monoxide according to any one of claims 1 to 6, which comprises process step (C):
(C) contacting the composition C-1 comprising dinitrogen monoxide with a gas mixture M-1 to obtain a composition C-2 and a gas mixture M-2.

8. The process for purifying a gas mixture comprising dinitrogen monoxide according to claim 7, wherein the gas mixture M-1 is selected from the group consisting of nitrogen, helium, neon, argon, krypton, xenon, hydrogen, carbon monoxide, methane and tetrafluoromethane.

9. The process for purifying a gas mixture comprising dinitrogen monoxide according to either of claims 7 and 8, wherein the gas mixture M-2 is recycled into step (A).

10. The process for purifying a gas mixture comprising dinitrogen monoxide according to any one of claims 7 to 9, wherein the gas mixture M-2 is recycled into step (i) or into step (iii) of step (A).

## Revendications

1. Procédé de purification d'un mélange gazeux G-0 contenant du monoxyde de diazote, comprenant au moins les étapes suivantes :
(A) le traitement du mélange gazeux G-0 contenant du monoxyde de diazote pour obtenir un mélange gazeux G-A, comprenant au moins les étapes suivantes :
(i) l'absorption du mélange gazeux G-0 dans un mélange de solvants LM-I pour obtenir un courant de gaz d'échappement et une composition Z-A,
(ii) la désorption d'un mélange gazeux G-1 de la composition Z-A pour obtenir un mélange de solvants LM-I',
(B) la condensation du mélange gazeux G-A pour obtenir une composition liquide Z-1 contenant du monoxyde de diazote et un mélange gazeux G-K,
le mélange gazeux G-K étant recyclé dans le traitement selon l'étape (A).

2. Procédé de purification d'un mélange gazeux contenant du monoxyde de diazote selon la revendication 1, dans lequel l'étape (A) comprend également les étapes (iii) et (iv) :
(iii) l'absorption du mélange gazeux G-1 dans un mélange de solvants LM-II pour obtenir un courant de gaz d'échappement et une composition Z-B,
(iv) la désorption d'un mélange gazeux G-2 de la composition Z-B pour obtenir un mélange de solvants LM-II'.

3. Procédé de purification d'un mélange gazeux contenant du monoxyde de diazote selon l'une quelconque des revendications 1 ou 2, dans lequel l'étape (A) comprend des étapes supplémentaires.

4. Procédé de purification d'un mélange gazeux contenant du monoxyde de diazote selon l'une quelconque des revendications 1 à 3, dans lequel le mélange gazeux G-K est recyclé dans l'étape (i) ou dans l'étape (iii) de l'étape (A).

5. Procédé de purification d'un mélange gazeux contenant du monoxyde de diazote selon l'une quelconque des revendications 1 à 4, dans lequel le mélange de solvants LM-I ou le mélange de solvants LM-II ou le mélange de solvants LM-I et le mélange de solvants LM-II sont choisis parmi le groupe constitué de solvants organiques et de mélanges de solvants aqueux.

6. Procédé de purification d'un mélange gazeux contenant du monoxyde de diazote selon l'une quelconque des revendications 1 à 5, dans lequel dans l'étape (B), de 20 à 90 % en poids du mélange gazeux G-A sont condensés.

7. Procédé de purification d'un mélange gazeux contenant du monoxyde de diazote selon l'une quelconque des revendications 1 à 6, le procédé comprenant l'étape (C) :
(C) la mise en contact de la composition Z-1 contenant du monoxyde de diazote avec un mélange gazeux S-1 pour obtenir une composition Z-2 et un mélange gazeux S-2.

8. Procédé de purification d'un mélange gazeux contenant du monoxyde de diazote selon la revendication 7, le mélange gazeux S-1 étant choisi dans le groupe constitué par l'azote, l'hélium, le néon, l'argon, le krypton, le xénon, l'hydrogène, le monoxyde de carbone, le méthane et le tétrafluorométhane.

9. Procédé de purification d'un mélange gazeux contenant du monoxyde de diazote selon l'une quelconque des revendications 7 ou 8, dans lequel le mélange gazeux S-2 est recyclé dans l'étape (A).

10. Procédé de purification d'un mélange gazeux contenant du monoxyde de diazote selon l'une quelconque des revendications 7 à 9, dans lequel le mélange gazeux S-2 est recyclé dans l'étape (i) ou dans l'étape (iii) de l'étape (A).
